# EUROPEAN PATENT APPLICATION

(11) **EP 2 957 629 A1**
(43) Date of publication of application: **23.12.2015**
(21) Application number: 14305939.2
(22) Date of filing: 18.06.2014
(51) Int. Cl.: C12N 1/32, C12N 15/52, C12P 7/24

(54) **Improved production of vanilloids by fermentation**

(71) Applicant: Rhodia Opérations, 75009 Paris (FR)
(72) Inventor: Ramaen, Odile, 78660 Ablis (FR); Sauveplane, Vincent, 78990 Elancourt (FR); Pandjaitan, Rudy, 94700 Maisons-Alfort (FR); Gelo-Pujic, Mirjana, 69360 Serezin-du-Rhône (FR)
(74) Representative: Nony

(57) **Abstract**

The present invention concerns a recombinant yeast comprising a set of exogenous nucleic acids, with each nucleic acid encoding a polypeptide involved in the biosynthesis pathway of a vanilloid, said set of exogenous nucleic acids encoding a set of polypeptides selected in a group comprising:
• a set of enzymes allowing conversion of an aromatic amino acid into a vanilloid; and
• a set of enzymes allowing conversion of a dehydroshikimic acid into a vanilloid;

and wherein said recombinant yeast is devoid of a functional gene adh6 encoding for an alcohol dehydrogenase,
and wherein said recombinant yeast has a reduced ability to decarboxylate phenylacrylic acids, which encompasses that said yeast does not comprise a fonctional *fdc1* gene and/ or a functional *pad1* gene.

## Description

### BACKGROUND

Vanilloids are defined as chemical compounds possessing a vanillyl group whose chemical structure is shown below: 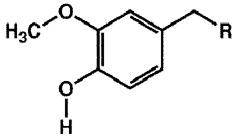 wherein R is selected from the group consisting of an hydrogen H, a methyl-CH₃, an ethyl -CH₂CH₃, an aldehyde and a carboxylic acid. Optionally, the OH group can be substituted with an O-glycosyl group. In a non-limitative manner, vanilloids include vanillyl alcohol, vanillin, vanillic acid, vanillin β-D-glucoside, acetovanillon, vanillylmandelic acid, homovanillic acid and capsaicin.

By extension, vanilloids also preferably includes chemical compounds possessing a group whose chemical structure is shown below: 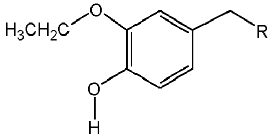 wherein R is selected from the group consisting of an hydrogen H, a methyl - CH₃, an ethyl -CH₂CH₃, an aldehyde and a carboxylic acid. Optionally, the OH group can be substituted with an O-glycosyl group. In a non-limitative manner, these chemical compunds include ethylvanillyl alcohol, ethylvanillin, ethylvanillic acid, ethylvanillin β-D-glucoside, acetoethylvanillon, ethylvanillylmandelic acid and homoethylvanillic acid.

Among vanilloids, vanillin, of chemical name 4-hydroxy-3-methoxybenzaldehyde, is one of the most important aromatic flavor compound used in foods, beverages, fragrances, pharmaceuticals and polymers. Vanillin was historically extracted from *Vanilla planifolia, Vanilla tahitiensis* and *Vanilla pompona* pods. The demand getting higher today, less than 5% of worldwide vanillin production comes from natural vanilla pods. Currently, chemical synthesis is the most important process for producing vanillin. There is a growing interest in other sources of vanillin and in particular in bioconversion processes. The use of microbial cells and their enzymes as biocatalysts for the synthesis of chemicals and flavor compounds have attracted much attention lately. Advantageously, under certain criteria, the products of such bioconversion may be considered 'natural' by legislations, such as the EU one.

Bioconversion processes are based on the following substrates: lignin, phenolic stilbenes, isoeugenol, eugenol, ferulic acid, sugars, phenolic stilbenes, waste residues and aromatic amino acids. The recent review from Kaur and Chakraborty (Kaur B, Chakraborty D. "Biotechnological and molecular approaches for vanillin production: a review." Appl Biochem Biotechnol. 2013 Feb; 169(4):1353-72) list several biosynthetic pathways and appropriate cells used for bioconversion of vanilloids.

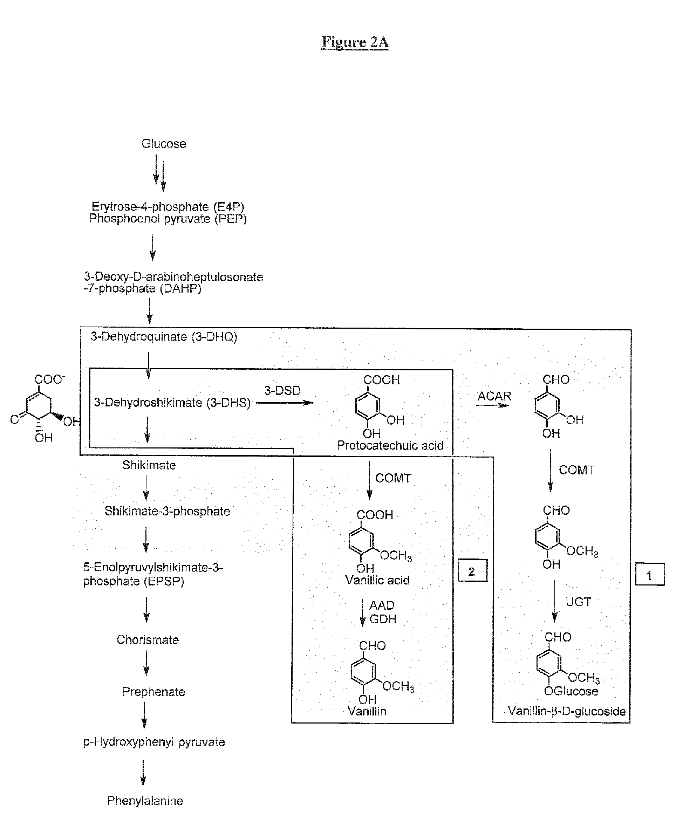

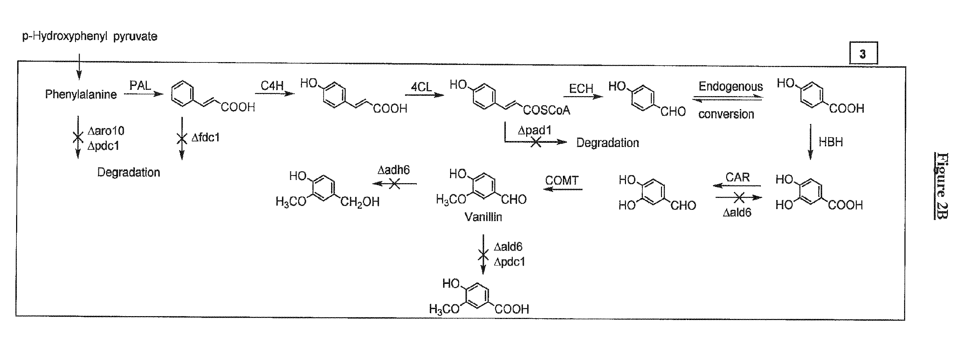

*De novo* synthesis from glucose and using metabolically engineered yeast strains was recently described (Hansen EH, Moller BL, Kock GR, Bünner CM, Kristensen C, Jensen OR, Okkels FT, Olsen CE, Motawia MS, Hansen J. "De novo biosynthesis of vanillin in fission yeast (Schizosaccharomyces pombe) and baker's yeast (Saccharomyces cerevisiae)". Appl Environ Microbiol. 2009 May;75(9):2765-74). The engineered pathway involves a 3-dehydroshikimate dehydratase (3DSD), an aromatic carboxylic acid reductase (ACAR) and an O-methyltransferase, as shown in figure 2, pathways 1 and 2. In S. *cerevisiae,* because the ACAR enzyme requires an activation mediated by a phosphopantetheinyl transferase, the latter enzyme was also introduced in the yeast. Prevention of reduction of vanillin to vanillyl alcohol was achieved by deleting the host alcohol dehydrogenase encoding gene *adh6.* Later, the same authors improved their engineered pathway by using mutants of human Catechol O-methyltransferase having better substrate specificity, thereby limiting the production of isovanillin (WO 2013/022881).

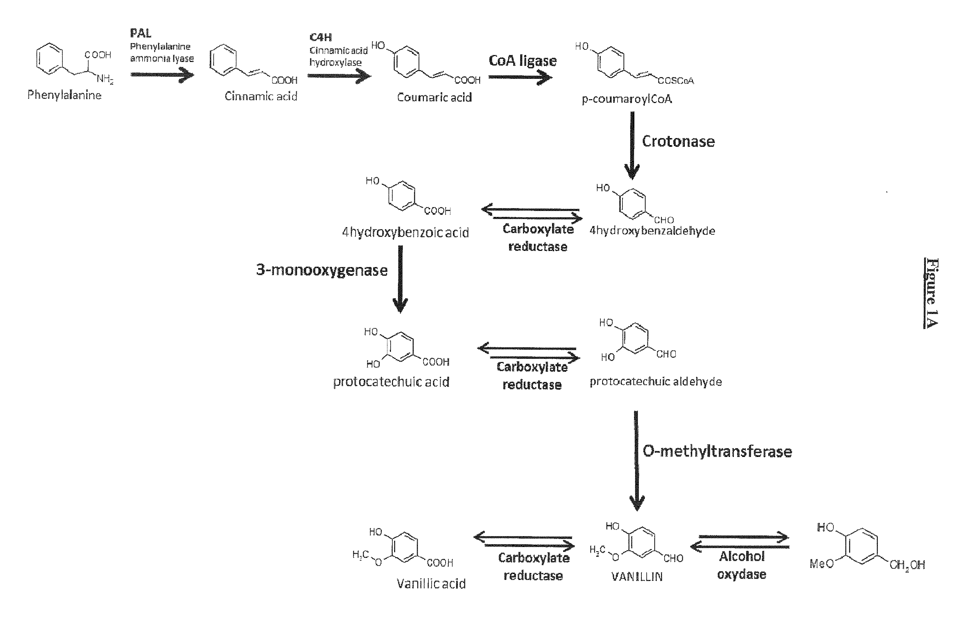

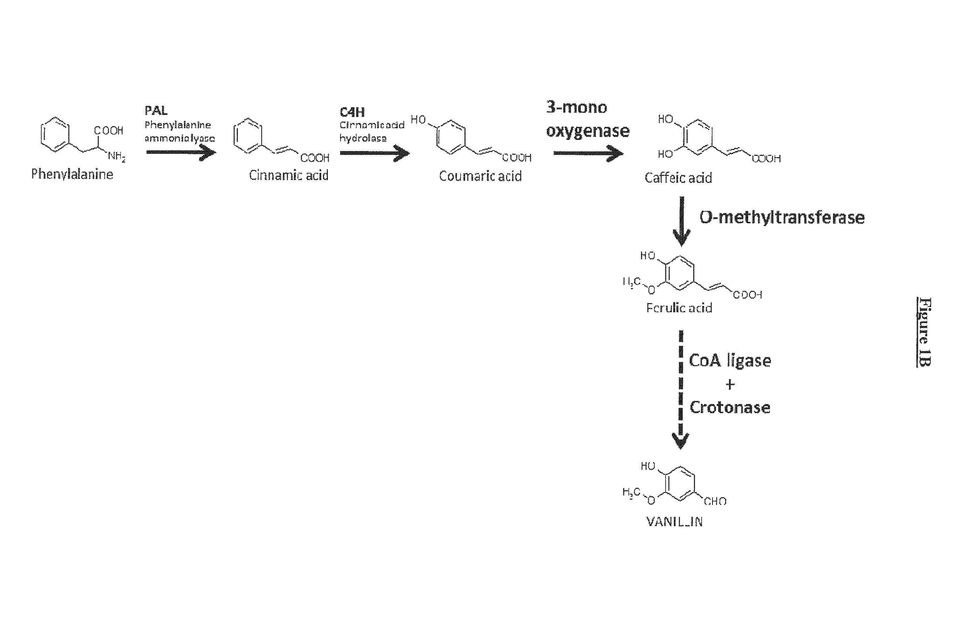

Another engineered pathway for producing a vanilloid in yeast is inspired from the natural biosynthesis pathway observed in the *Vanilla planifolia* orchid, such as described in patent application US 2003/0070188. This engineered pathway comprises the following enzymes: phenylalanine/tyrosine ammonia lyase (PAL/TAL), cinnamate-4-hydrolase (C4H), 4-coumarate coenzyme A ligase (4CL), enoyl-CoA hydratase/aldolase (ECH), a hydroxybenzoic acid hydroxylase (HBH) and a caffeic acid O-methyltransferase (COMT), as shown in figures 1 and 2, pathway 3. This pathway uses aromatic amino acids such as phenylalanine and tyrosine as primary substrates. Enzymes of this pathway have been successfully introduced into yeast, able to bio-synthetize vanilloids (patent application EP13156021.1, not published, herein incorporated by reference).

However, even if these engineered pathways are effective for producing vanilloids, they do not allow production of these compounds in amounts which are compatible with the economical and industrial requirements. Different propositions have been made to improve the production of vanilloids by fermentation in cells.

Among them, alcohol dehydrogenases ADH6 & ADH7 are known to convert vanillin into its corresponding vanillyl alcohol. Studies have suggested the deletion of the *adh6* gene in vanillin-producing yeasts (Hansen *et al.,* 2009, previously cited).

The article from Brochado *et al.* (Brochado AR, Matos C, Moller BL, Hansen J, Mortensen UH, Patil KR. "Improved vanillin production in baker's yeast through in silico design". Microb Cell Fact. 2010 Nov 8;9:84) suggested the deletion of genes encoding pyruvate decarboxylase (PDC1) and glutamate dehydrogenase (GDH1), since the deletion of these genes increases the respiratory capacity of the cell and the availability of co-factors (ATP, NADPH...) for the biosynthesis pathway of vanilloids.

Most of phenolic compounds such as vanillin show some toxicity for many living organisms with increased concentration of vanilloids. In case of *Saccharomyces cerevisiae,* growth defect is significant with concentrations as low as 0.5 g/l. To avoid the impaired growth of producing microorganisms, it has been proposed to isolate the produced vanilloids from the culture medium, in particular with resins. Another suggested solution is promoting conversion of vanillin into vanillin β-D-glucoside, this glycosylation inhibiting its toxic effect (Hansen et al., 2009; Brochado et al., 2010, both previously cited).

However, the highest amount of vanillin produced in bioconversion process with these genetically modified microorganisms is still limited to about 1 µM; indeed several problems subsist and have to be solved to improve vanillin production. Specific disadvantages linked to bioconversion processes are in particular the following:
• In living cells, intermediates of the biosynthesis pathway are subject to degradation by endogenous enzymes. In particular, phenylacrylic acids such as cinnamic acid and coumaric acid can be decarboxylated by endogenous yeasts enzymes and converted into styrene and 4-vinylphenol. Also, aldehydes compounds in the pathway are subject to reduction in their corresponding primary alcohol by various alcohol dehydrogenases. Finally, the aromatic amino acids, first intermediates of the pathway, are also subject to consumption in concurrent metabolic pathways.
• Efficiency of bioconversion is linked to the growth ability of the producing cells; therefore, it is essential to determine the best conditions of growth for said producing cells. Different parameters can be modified, in order to optimize the growth of cells and therefore the metabolic pathways, for example: volume of the culture, temperature, growth medium, source of carbon, management of the toxic effect of vanillin, etc.

Inventors present here genetically modified yeast cells and specific culture conditions, both allowing a significant improvement of vanilloid production from a source of carbon, by fermentation.

### SUMMARY OF THE INVENTION

The present invention is related to a genetically modified yeast, optimized for the production of a vanilloid or a precursor thereof from a source of carbon, comprising enzymes required for production of a vanilloid or a precursor thereof, and wherein the said genetically modified yeast has a reduced ability to decarboxylate phenylacrylic acids. Advantageously, the genetically modified yeast of the invention presents also a decreased ability for degrading aldehydes, and ensures an improved availability of aromatic amino acids.

The present invention is also related to an improved method for producing a vanilloid or a precursor thereof by fermentation. Culture conditions such as carbon sources, biphasic media bioconversion and volume of the culture were explored to optimize vanillin production.

The present invention is also related to a method for producing a vanilloid or a precursor thereof from a specific source of carbon, by fermentation of genetically modified yeasts.

The present invention is also related to a method for producing a vanilloid or a precursor thereof, and separating it from the culture medium by biphasic culture media to avoid the toxic effect of vanillin. In particular the step of the yeast culture is performed in an appropriate medium comprising two phases, an aqueous phase and a water immiscible solvent.

In addition, the present invention is related to a composition comprising a vanilloid or a precursor thereof obtainable by the method as disclosed above, and to the use of said composition as a flavoring in the human and animal nutrition field, in pharmacy, and as a fragrance in the cosmetics, perfumery and detergency industries.

### FIGURES

FIGURE 1A, 1B: Biosynthetic pathways of vanillin from aromatic amino acids. The figures show the schematic diagram wherewith carbon source is converted into vanillin. Phenylalanine undergoes several reactions: deamination, hydroxylation of 4 position of the phenyl ring, reduction chain reaction and hydroxylation of 3 position of the phenyl ring, and O-methylation of the 3 position of the phenyl ring.
FIGURE 2A, 2B: Global reaction scheme of vanillin biosynthesis from glucose. The shikimic acid pathway (pathways 1 & 2 - Figure 2A) and the aromatic amino acid pathway (pathway 3 - Figure 2B) are represented. PAL relates to a phenylalanine ammonia lyase. C4H relates to a cinnamic acid hydroxylase. 4CL relates to a 4-coumarate-CoA ligase. HBH relates to a hydroxybenzoic acid hydroxylase. ECH relates to an enoyl-CoA hydratase / crotonase.

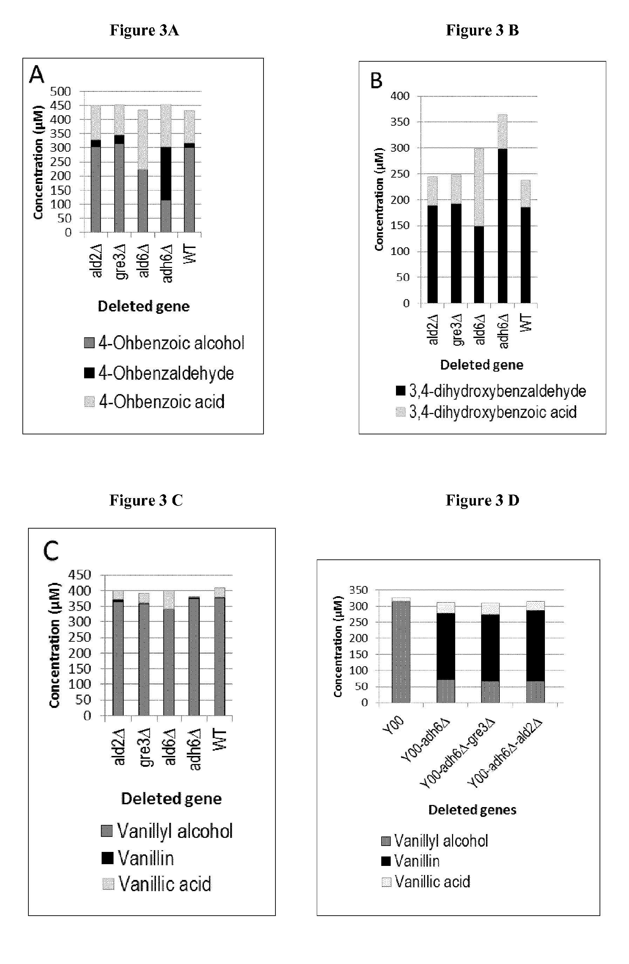

FIGURE 3: 3A: conversion of 4-hydroxybenzaldehyde by deletion strains Δald2, Δgre3, Δald6 and Δadh6 and a wild-type (WT) strain; 3B: conversion of 3,4-dihydroxybenzaldehyde by the same strains than figure 3A; 3C: conversion of vanillin by the same strains than figure 3A; 3D: conversion of vanillin by the strain Y00, Y00 Δadh6 and the multi-deleted strains Y00 Δadh6 Δgre3 and Y00 Δadh6 Δald2.

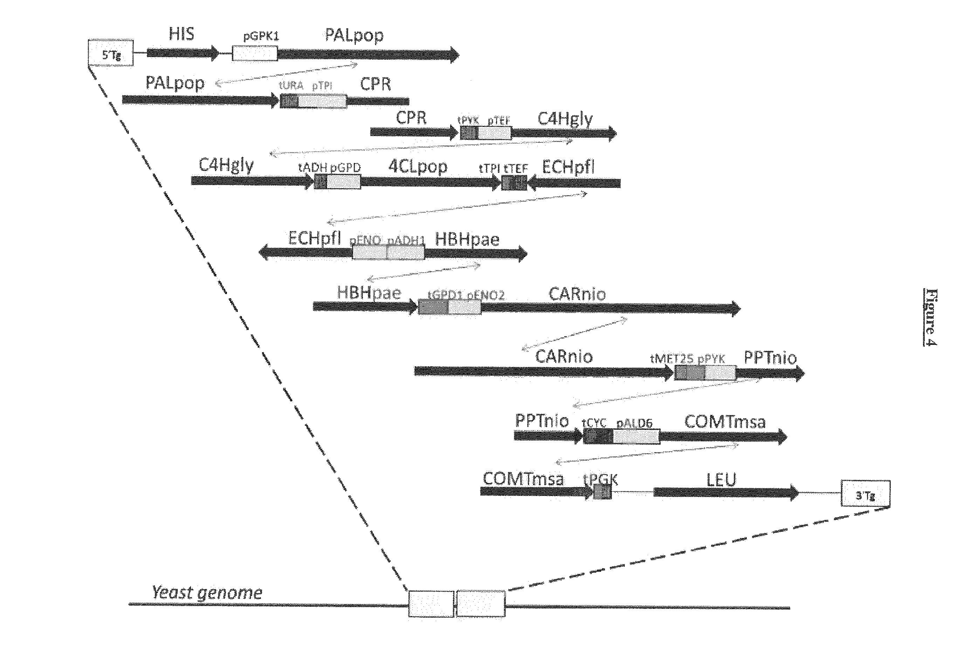

FIGURE 4: Assembly of vanillin pathway by fragments containing homologous gene sequences. This figure shows the co-transformation of 9 fragments comprising the 6 genes for vanillin production starting from phenylalanine and 1 gene coding for the carboxylic acid reductase. HIS3 and LEU2 are the flanking markers enabling the double selection of the recombinant pathway.

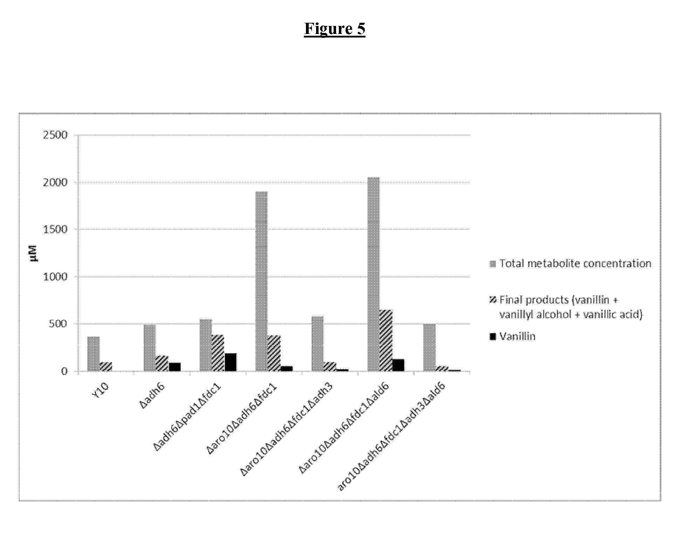

FIGURE 5: Grey bars: Metabolites total concentration from vanillin pathway according to then host strain (cinnamic acid, coumaric acid, 4-hydroxybenzoic acid, 3,4-dihydroxybenzoic acid, 3,4-dihydroxybenzaldehyde, vanillin, vanillic acid, vanillyl alcohol). Striped bars: Total concentration of final products from vanillin pathway according to then host strain (vanillin, vanillic acid and vanillyl alcohol). Black bars: vanillin concentration.

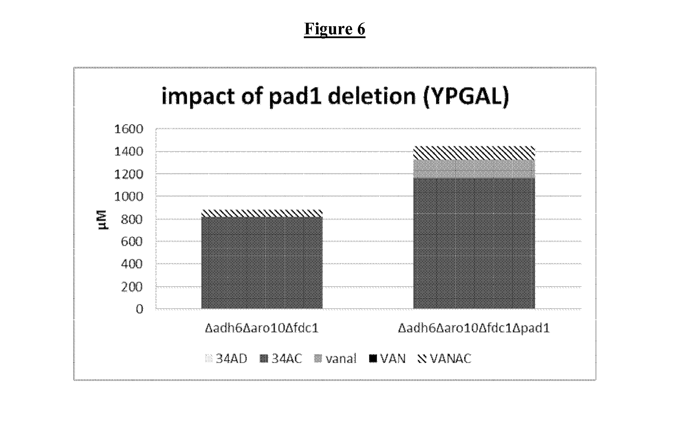

FIGURE 6: Metabolite profile of supernatant of culture from strains comprising a recombinant pathway according to figure 4, and the following deletions: Δadh6Δaro10Δfdc1 and Δadh6Δaro10Δfdc1Δpdc1 in 2mL culture volume. 34AD = 3,4-dihydroxybenzaldehyde; 34AC = 3,4-dihydroxybenzoic acid; VANAL = vanillyl alcohol; VAN = vanillin; VANAC = vanillic acid.

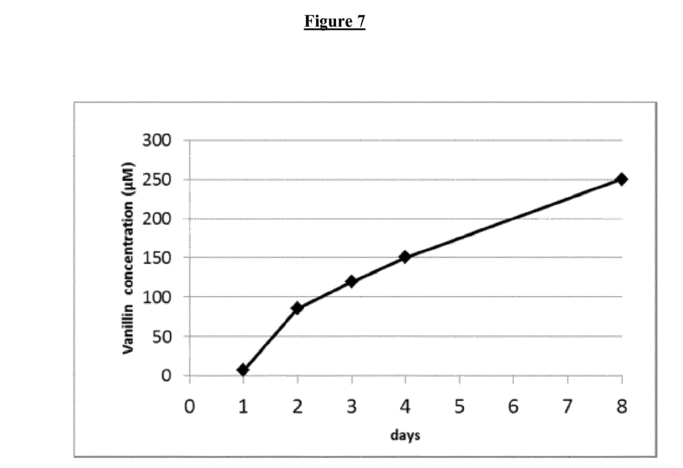

FIGURE 7: Vanillin production of a strain comprising a recombinant pathway according to figure 4, and the following deletions: Δadh6Δfdc1Δpad1 in a 50 mL Erlenmeyer flask.

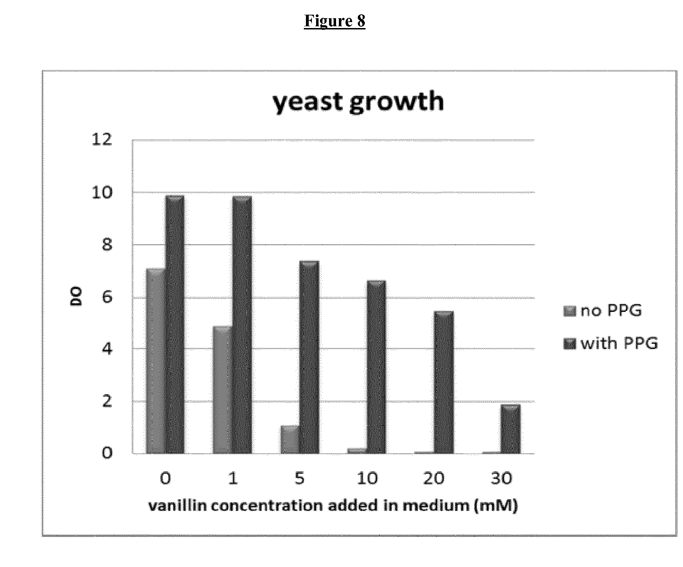

FIGURE 8: Cytotoxicity assays with vanillin toward yeast in the presence or absence of PPG1200 done with a strain comprising a recombinant pathway according to figure 4, and the following deletions: Δadh6Δpdc1Δpad1.

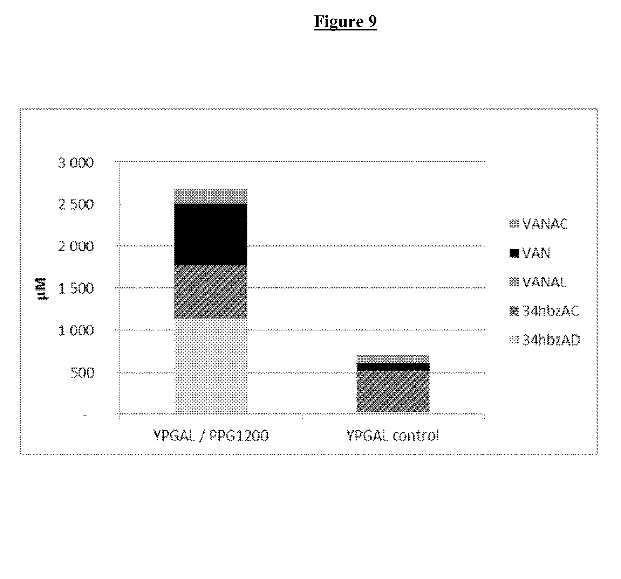

FIGURE 9: Extractive bioconversion of vanillin using 30% polypropylene glycol 1200 (PPG). Diagram presents concentration of vanillin (VAN), vanillic acid (VANAC), vanillyl alcohol (VANAL), 3,4-dihydroxybenzoic acid (34hbzAC) and 3,4-dihydroxybenzaldehyde (34hbzAD) in control culture (YPGal) and in biphasic culture (YPAGal/PPG1200) from culture supernatant of a strain comprising a recombinant pathway according to figure 4, and the following deletions: Δadh6Δpad1Δfdc1.

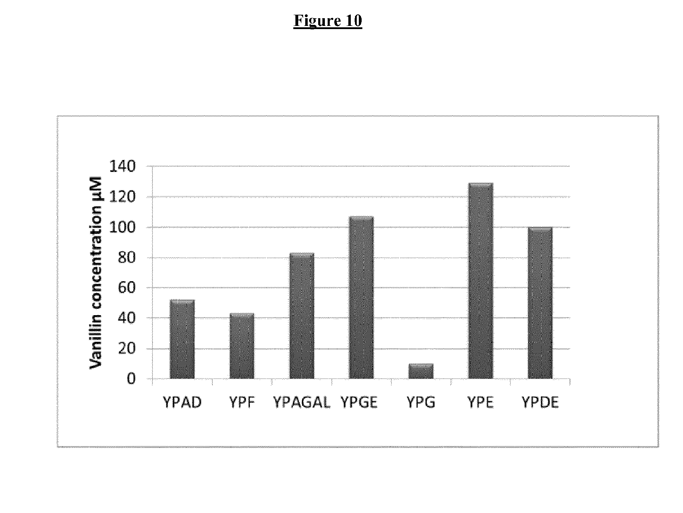

FIGURE 10: Vanillin producing cells (strains comprising a recombinant pathway according to figure 4, and the following deletions: Δadh6Δpdc1Δfdc1) were precultured in YPD (10 g/l bacto-yeast extract, 20 g/l bacto-peptone and 2% dextrose) or YPGal (10 g/l bacto-yeast extract, 20 g/l bacto-peptone and 3% galactose) medium in a 10 mL for 24h, and used to inoculate 50 mL appropriate medium in a 250 mL Erlenmeyer flask. Vanillin concentration (µM) was monitored after 3 days of culture depending on carbon source used for yeast growth. YPAD = YPGal; YPF (10 g/l bacto-yeast extract, 20 g/l bacto-peptone and 2% fructose); YPAGal (10 g/l bacto-yeast extract, 20 g/l bacto-peptone, 30g/l galactose); YPGE (10 g/l bacto-yeast extract, 20 g/l bacto-peptone, 2% glycerol and 2% ethanol); YPG (10 g/l bacto-yeast extract, 20 g/l bacto-peptone, 2% glycerol); YPE (10 g/l bacto-yeast extract, 20 g/l bacto-peptone and 2% ethanol); YPDE (10 g/l bacto-yeast extract, 20 g/l bacto-peptone, 2% dextrose and 2% ethanol).

### DETAILLED SPECIFICATION

The present invention is related to a recombinant yeast, optimized for the production of a vanilloid or a precursor thereof, comprising a set of exogenous nucleic acids, with each nucleic acid encoding a polypeptide involved in the biosynthesis pathway of a vanilloid, said set of exogenous nucleic acids encoding a set of polypeptides selected in a group comprising:
- a set of enzymes allowing conversion of an aromatic amino acid into a vanilloid; and
- a set of enzymes allowing conversion of an dehydroshikimic acid into a vanilloid;
and wherein said recombinant yeast is devoid of a functional gene *adh6* encoding for an alcohol dehydrogenase,
and wherein said recombinant yeast has a reduced ability to decarboxylate phenylacrylic acids, which encompasses preferentially that said yeast does not comprise a fonctional *fdc1* gene and/ or a functional *pad1* gene.

This invention also pertains to a recombinant yeast, optimized for the production of a vanilloid or a precursor thereof, expressing a set of polypeptides involved in the biosynthesis pathway of a vanilloid, said set of polypeptides being encoded by one or more exogenous nucleic acids, and said set of polypeptides being selected in a group comprising:
- a set of enzymes allowing conversion of an aromatic amino acid into a vanilloid; and
- a set of enzymes allowing conversion of an dehydroshikimic acid into a vanilloid;
and wherein said recombinant yeast is devoid of a functional gene *adh6* encoding for an alcohol dehydrogenase,
and wherein said recombinant yeast has a reduced ability to decarboxylate phenylacrylic acids, which encompasses that said yeast does not comprise a functional *fdc1* gene and/ or a functional *pad1* gene.

The following terms are used in the present application and are defined hereunder.

Yeasts are eukaryotic unicellular microorganisms. Yeasts are chemoorganotrophs, as they use organic compounds as a source of energy and do not require sunlight to grow. Over 1,500 species are currently known. An extensively studied genus of yeast is *Saccharomyces.*

By "recombinant yeast" or "genetically modified yeast" is meant a yeast organism whose genetic material has been modified by genetic engineering methods, either by suppression or inactivation of genes, and/or by addition of exogenous genetic material.

By "vanilloid" is meant a chemical compound which possesses a vanillyl group whose chemical structure is shown below: 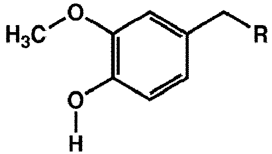 wherein R is selected from the group consisting of H, -CH₃, -CH₂CH₃, an aldehyde and a carboxylic acid. Optionally, the OH group can be substituted with an O-glycosyl group. By extension, vanilloids also preferably include chemical compounds possessing a group whose chemical structure is shown below: 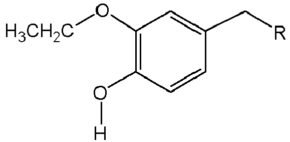 wherein R has the same meaning as defined above.

The present invention relates to a method for producing a vanilloid of formula (I) or of formula (II), preferably of formula (I):

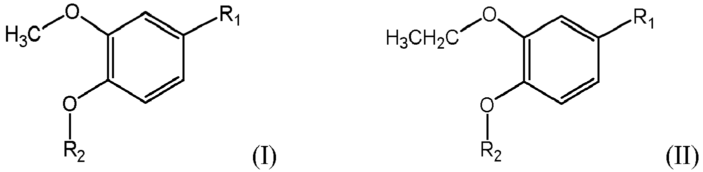

R¹ being selected from the group consisting of -CHO; -COOH; -COOR³ - CH₂OH; -CH₂COOH; -C(=O)CH₃; -CR³(OH)COOH and CHR³COOH, wherein R³ is a lower alkyl,

R² being different from a methyl (-CH₃), in particular R² being selected from the group consisting of H, sulfate, phosphate and a glycoside. According to a preferred embodiment, R² can be H.

Vanilloids include, in a non limitative manner, vanillyl alcohol, vanillin, vanillic acid, vanillin-glycoside, acetovanillon, vanillylmandelic acid, homovanillic acid, capsaicin and ethylvanillin.

Chemical structures of vanillin, isovanillin, vanillic acid and isovanillic acid are respectively given here below:

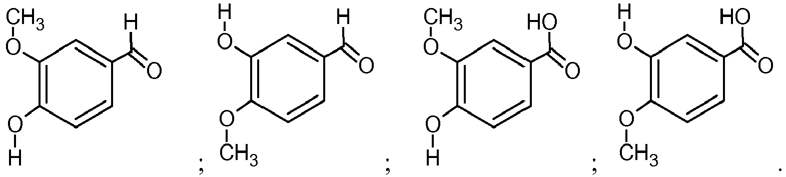

As used herein, an "exogenous nucleic acid encoding a polypeptide" means a nucleic acid comprising a one or more expression regulatory sequences and an Open Reading Frame (which may also be termed "ORF" or "coding sequence"), that has been initially introduced into a yeast cell by a genetic engineering method, thus allowing the said yeast cell to express a polypeptide encoded by the said ORF. Most preferably, an exogenous nucleic acid also comprises at least a transcription terminator. Nucleic acids are exogenous to the host cell, i.e. are introduced in said host cell; they can be naturally absent or present in said host cell, but in all cases they are introduced by human manipulation. Most preferably, an "exogenous nucleic acid encoding a polypeptide" is not naturally present in the genome of the said yeast cell. An example of an exogenous nucleic acid is a nucleic acid which originates from a yeast species different from the host yeast cell species. Another example of an exogenous nucleic acid is a construct comprising an endogenous ORF which is placed under the control of a promoter which is not the naturally-occurring promoter of the said endogenous ORF.

As used herein, a "set" of exogenous nucleic acids means a plurality of exogenous nucleic acids as defined above, the said plurality of exogenous nucleic acids allowing when expressed in yeast cells wherein they have been introduced, the production of the corresponding set of polypeptides. The examples herein illustrate embodiments of yeast cells in which a set of exogenous nucleic acids has been introduced, the said set of exogenous nucleic acids encoding a set of polypeptides selected in a group comprising:
- a set of enzymes allowing conversion of an aromatic amino acid into a vanilloid; and
- a set of enzymes allowing conversion of an dehydroshikimic acid into a vanilloid.

In some embodiments, a set of exogenous nucleic acids is comprised in a unique polynucleotide, which unique polynucleotide has been introduced in the yeast organism, e.g. within a specific location in a yeast chromosome.

In other embodiments, a set of exogenous nucleic acids is initially comprised in a plurality of polynucleotides which are then fused before introduction in the yeast organism, e.g. within a specific location in a yeast chromosome.

In still other embodiments, a set of exogenous nucleic acids is comprised in a plurality of polynucleotides, each polynucleotide being introduced in the yeast organism, e.g. within a plurality of locations in a yeast chromosome.

As used herein, a recombinant yeast organism "comprises" exogenous nucleic acids when the said exogenous nucleic acids are part of the genetic material thereof, either (i) because the said exogenous nucleic acids are integrated into the chromosomal genetic material of the said yeast organism, (ii) or because the said exogenous nucleic acids are included within the genetic material of the said yeast organism outside chromosomes, which encompasses inclusion of the exogenous nucleic acids into nucleic acid vectors, e.g. plasmids. The term "biosynthesis pathway" as used in the present application refers to the different enzymes involved in specific biochemical steps and intermediates enabling the biosynthesis of products from a substrate *in vivo* by recombinant host cells. A biosynthesis pathway may be termed "artificial" when one or more of the enzymes comprised therein are encoded by exogenous nucleic acids, i.e. nucleic acids that have been introduced 'artificially' into a host cell, most preferably by using genetic engineering methods.

The term "aromatic amino acid" designates an amino acid that includes an aromatic ring. Among the twenty standard amino acids, four of them consist of aromatic amino acids: phenylalanine, tryptophan, histidine and tyrosine.

The term "dehydroshikimic acid" also called 3-DHS designates the compound of the systemic name (4S,5R)-4,5-D-dihydroxy-3-oxocyclohexene-1-carboxylic acid, whose complete formula is shown in figure 2.

### Recombinant yeasts with genetic modifications optimizing the synthesis of vanilloids

Different genetic modifications are proposed for optimizing the biosynthesis of vanilloids in recombinant yeasts comprising exogenous nucleic acids coding for polypeptides involved in the biosynthesis of vanilloids.

As previously reported, prevention of the reduction of vanillin into vanillyl alcohol can be achieved by the inactivation or the deletion of at least one gene coding for host alcohol dehydrogenases, in particular by the inactivation or the deletion of the gene *adh6.*

In the present invention, the recombinant yeast is devoid of a functional gene *adh6.*

The expression "a functional gene is absent" or "said yeast does not comprise a functional gene" or "said yeast is devoid of a functional gene" means that the said gene does not allow the production of the expected functional polypeptide, e.g. does not allow the production of the expected active enzyme. In some embodiments of a yeast cell that is devoid of a functional given gene, the said gene is not expressed in the yeast cell, e.g. the said gene is not transcribed or the corresponding transcription product is not translated into a protein, e.g. into an enzyme, having its usual activity. In some embodiment, the said gene may be 'inactivated'. The man skilled in the art knows various means to obtain such absence of functionality/inactivation of a gene, such as:
- introduction of a mutation into the gene, in particular generation of a stop codon inducing the expression of a non-functional, truncated protein;
- introduction of an 'insert' into the gene, inactivating its correct transcription; e.g. interruption of the gene sequence by introduction of one or more exogenous nucleic acids, which encompasses introduction of a set of exogenous nucleic acids,
- replacement of the natural promoter of the gene by a non-functional promoter, or complete suppression of the promoter,
- deletion, complete or partial of the coding sequence of the gene.

In some preferred embodiments, "absence of a functional gene" refers to an endogenous gene having been inactivated by introduction of a DNA insert, or refers to a gene whose coding sequence has been partially of completely deleted.

Phenylacrylic acids include cinnamic acid, coumaric acid and ferulic acid, all of them present in numerous plants. These carboxylic acids are intermediates in the pathway of catabolism of aromatic amino acids. Mukai *et al.* have shown that genes encoding PAD1 and FDC1 are essential for the decarboxylation of phenylacrylic acids in *S. cerevisiae* (Mukai N, Masaki K, Fujii T, Kawamukai M, Iefuji H. "PAD1 and FDC1 are essential for the decarboxylation of phenylacrylic acids in Saccharomyces cerevisiae." J Biosci Bioeng. 2010 Jun;109(6):564-9).

The expression "reduced ability to decarboxylate phenylacrylic acids" means, for the purpose of the present description, that the intermediates in the vanilloid biosynthesis pathway, such as cinnamic acid, coumaric acid, and ferulic acid, are less converted by decarboxylases than in a wild-type corresponding yeast, and therefore that the availability of these compounds for the biosynthesis pathway of vanilloid is increased. According to the invention, the yeast cell is genetically modified so as to possess a low capacity of decarboxylating phenylacrylic acids, because of the absence of at least one functional gene coding for the decarboxylases Pad1p and/or Fdclp. Respective contribution of the Pad1p and Fdclp proteins to the decarboxylation of phenylacrylic acids is disclosed in the article of Mukai et al. (2010), previously cited.

In a specific embodiment of the invention, the genetically modified yeast is further modified to decrease the consumption of aromatic amino acids in other biosynthesis pathways than the vanilloid biosynthesis pathway. In particular, the expression and/or activity of enzymes using aromatic amino acids as substrates could be decreased, except for the enzymes phenylalanine ammonia lyase and tyrosine ammonia lyase.

Advantageously, the genetically modified yeast does not comprise a functional gene *aro10. Aro10* gene encodes a phenylpyruvate decarboxylase that is involved in the consumption of phenylalanine and tyrosine (Vuralhan Z, Morais MA, Tai SL, Piper MD, Pronk JT. « Identification and characterization ofphenylpyruvate decarboxylase genes in Saccharomyces cerevisiae » Appl Environ Microbiol. 2003 Aug; 69(8):4534-41). The disruption of *aro10* causes an improved availability of phenylalanine for the biosynthesis pathway towards vanilloids.

In vanillin pathway, many intermediate precursors are aldehydes, which are known to be substrates of many endogenous enzymes leading to their corresponding alcohol or acid derivative. In recombinant yeasts according to the invention, aldehydes tend to be oxidized immediately after the reduction chain reaction, and as a consequence no vanillin is detected. In order to lower the conversion of aldehydes into their corresponding acid derivatives, a carboxylic acid reductase protein may be added, that is able to convert aromatic carboxylic acid into corresponding aldehyde. The CAR enzyme requires activation by phosphopantetheinylation (this post-translation modification is done by PPTase).

In a preferred embodiment of the invention, the genetically modified yeast does not comprise a functional gene *ald6,* coding for an aldehyde dehydrogenase. Its disruption causes an improved availability of aldehyde intermediates in the biosynthesis pathway towards vanilloids.

Advantageously, the genetically modified yeast is further devoid of a functional gene *pdcl. Pdc1* gene encodes a pyruvate decarboxylase, converting pyruvate into acetaldehyde. Pyruvate is a key metabolite in *S. cerevisiae* metabolism and the branch point between respiratory and fermentation metabolism. It has been shown that deletion of *pdc1* results in a reduction of 30% of total pyruvate decarboxylase activity; leading to an increased production of vanillin (Brochado et al., 2010, previously cited).

In a specific embodiment, a recombinant yeast comprising a set of exogenous nucleic acids encoding a set of enzymes allowing conversion of an aromatic amino acid into a vanilloid, presents one of the following phenotypes: Δahd6Δfdc1, Δahd6Δfdc1Δaro10, Δahd6Δfdc1Δald6, Δahd6Δfdc1Δpdc1, Δahd6Δfdc1Δaro10Δald6, Δahd6Δfdc1 Δaro10Δpdc1, Δahd6Δfdc1Δa1d6Δpdc1, Δahd6Δfdc1Δaro10Δald6Δpdc1, Δahd6Δpad1, Δahd6Δpad1Δaro10, Δahd6Δpad1Δald6, Δahd6Apad1Δpdc1, Δahd6Δpad1Δaro10Δald6, Δahd6Δpad1Δaro10Δpdc1, Δahd6Δpad1Δald6Δpdc1, Δahd6Δpad1Δaro10Δald6Δpdc1, Δahd6Δfdc1Δpad1, Δahd6Δfdc1Δpad1Δaro10, Δahd6Δfdc1Δpad1Δald6, Δahd6Δfdc1Δpad1Δpdc1, Δahd6Δfdc1Δpad1Δaro10Δald6, Δahd6Δfdc1Δpad1Δaro10Δpdc1, Δahd6Δfdc1Δpad1Δal d6Δpdc1, Δahd6Δfdc1Δpad1Δaro10Δald6Δpdc1 or any other combination of these deletions.

In another specific embodiment, a recombinant yeast comprising a set of exogenous nucleic acids encoding a set of enzymes allowing conversion of an dehydroshikimic acid into a vanilloid, presents one of the following phenotypes: Δahd6Δfdc1, Δahd6Δfdc1Δaro10, Δahd6Δfdc1Δald6, Δahd6Δfdc1Δpdc1, Δahd6Δfdc1 Δaro10Δald6, Δahd6Δfdc1 Δaro10Δpdc1, Δahd6Δfdc1Δald6Δpdc1, Δahd6Δfdc1Δaro10Δald6Δpdc1, Δahd6Δpad1, Δahd6Δpad1Δaro10, Δahd6Δpad1Δald6, Δahd6Δpad1Δpdc1, Δahd6Δpad1Δaro10Δald6, Δahd6Δpad1Δaro10Δpdc1, Δahd6Δpad1Δald6Δpdc1, Δahd6Δpad1Δaro10Δald6Δpdc1, Δahd6Δfdc1Δpad1, Δahd6Δfdc1Δpad1Δaro10, Δahd6Δfdc1Δpad1Δald6, Δahd6Δfdc1Δpad1Δpdc1, Δahd6Δfdc1Δpad1Δaro10Δald6, Δahd6Δfdc1Δpad1Δaro10Δpdc1, Δahd6Δfdc1Δpad1Δal d6Δpdc1, Δahd6Δfdc1Δpad1Δaro10Δald6Δpdc1 or any other combination of these deletions.

### Enzymes involved in the biosynthesis pathway of vanilloids from aromatic amino acids

In the present description, enzymes are identified by their specific activities. This definition thus includes all polypeptides that have the defined specific activity also present in other organisms. Enzymes with similar activities can be identified by homology. Using the references given in GenBank for known genes, those skilled in the art are able to determine the equivalent genes in other organisms, bacterial strains, yeasts, plants, etc. This routine work is advantageously done using consensus sequences that can be determined by carrying out sequence alignments with genes derived from other microorganisms, and designing degenerate probes to clone the corresponding gene in another organism. These methods of molecular biology are well known to those skilled in the art, and are described, for example, in Green & Sambrook (Molecular Cloning: a Laboratory Manual. 4th ed. 2012 Cold Spring Harbor Lab., Cold Spring Harbor, New York).

According to a first embodiment of the invention, the artificial vanillin pathway uses endogenous aromatic amino acids (AAA) as primary substrates. Endogenously produced phenylalanine or tyrosine is diverted to the synthesis of vanillin. Exogenous phenylalanine and/or tyrosine can also be added into the culture medium of the yeast. Six enzymes are required for the conversion of L-phenylalanine/tyrosine to vanillin (see figures 1A and 1B).

In particular, phenylalanine is converted to coumarate by the successive actions of the enzymes phenylalanine ammonia lyase (PAL) and cinnamate-4-hydroxylase (C4H). The following step is the reduction chain reaction of the coumaric acid leading to the 4-hydroxybenzaldehyde. The reaction is initiated by the activation of coumaric acid to coumaroyl-CoA provided by 4CL enzyme (4-coumarate coenzyme A ligase), followed by a ß-oxidation performed by ECH enzyme (enoyl-CoA hydratase/aldolase, an enzyme of a crotonase family). The next step is the hydroxylation on 3-position of the phenyl ring carried out by HBH enzyme (hydroxybenzoic acid hydroxylase, 3-monooxygenase enzyme family). The final step is the 3-O-methylation leading to vanillin final product. This step is catalyzed by the COMT enzyme (caffeic acid O-methyltransferase, O-methyltransferase enzyme family). In order to lower endogenous conversion of aldehyde intermediate product, carboxylic acid reductase (CAR) protein is preferentially added. The CAR enzyme requires activation by phosphopantetheinylation, and this is achieved by co-expression of a phosphopantetheinyl transferase (PPTase).

According to a specific embodiment of the invention, the aromatic acid is phenylalanine, and the recombinant yeast comprises a set of exogenous nucleic acids coding for the following enzymes: phenylalanine ammonia lyase (PAL), cinnamate-4-hydrolase (C4H), 4-coumarate coenzyme A ligase (4CL), enoyl-coA hydratase/aldolase (ECH), a hydroxybenzoic acid hydroxylase (HBH) and a caffeic acid O-methyltransferase (COMT).

According to another specific embodiment of the invention, the aromatic amino acid is tyrosine, and the genetically modified yeast comprises a set of exogenous nucleic acids coding for the following enzymes: tyrosine ammonia lyase (TAL), cinnamate-4-hydrolase (C4H), 4-coumarate coenzyme A ligase (4CL), enoyl-CoA hydratase/aldolase (ECH), a hydroxybenzoic acid hydroxylase (HBH), and a caffeic acid O-methyltransferase (COMT).

The phenylalanine ammonia lyase (PAL) is an enzyme catalyzing the tyrosine deamination reaction classified EC 4.3.1.23. The tyrosine ammonia lyase (TAL) is an enzyme catalyzing the phenylalanine deamination reaction classified EC 4.3.1.24. Another enzyme is known for catalyzing the phenylalanine or tyrosine deamination reaction, classified EC 4.3.1.25, and called PAL/TAL. In a specific embodiment, the PAL enzyme is issued from *Petunia species* or *Populus species.* In another specific embodiment, a PAL/TAL enzyme from maize (Rösler, Krekel, Amrhein, Schmid. "Maize phenylalanine ammonia-lyase has tyrosine ammonia-lyase activity" Plant Physiol. 1997 January; 113(1): 175-179) is introduced into the recombinant yeast. This enzyme is able to convert phenylalanine into cinnamic acid and tyrosine into coumaric acid.

The cinnamic acid hydroxylase (C4H) is an enzyme catalyzing the cinnamate 4-hydroxylation, classified EC 1.14.13.11. It is a P450-dependent enzyme. In a specific embodiment, the C4H enzyme is issued from *Petunia species* or *Glycin species.*

The 4-coumarate coenzyme A ligase (4CL) is an enzyme catalyzing the CoA esterification of coumaric acid, classified EC 6.2.1.12. This enzyme belongs to the family of ligases, specifically those forming carbon-sulfur bounds as acid-thiol ligases. In a specific embodiment, the 4CL enzyme is issued from *Populus species.*

The enoyl-CoA hydratase/aldolase (ECH) is an enzyme catalyzing the hydratation of 2-trans-enoyl-CoA into 3-hydroxyacyl-CoA, belonging to the family of crotonases, and is classified EC 4.2.1.17. In a specific embodiment, the ECH enzyme is issued from *Pseudomonas fluorescens* or *Azotobacter vinelandii.*

The hydroxybenzoic acid hydrolase (HBH) is an enzyme catalyzing the hydroxylation of 4-hydroxybenzaldehyde. The HBH enzyme is also known as 4-hydroxybenzoate-3-monooxygenase (EC 1.14.13.2). In a specific embodiment, the HBH enzyme is issued from *Pseudomonas fluorescens* or *Azotobacter vinelandii.*

The caffeic acid O-methyltransferase (COMT) is an enzyme catalyzing the chemical conversion of 3,4-dihydroxy-trans-cinnamate (caffeic acid) to 3-methoxy-4-hydroxy-trans-cinnamate (ferulic acid). This enzyme is also capable of converting protocacheuic aldehyde to vanillin. This enzyme is classified EC 2.1.1.68 and belongs to the family of methyltransferases.

Preferentially, the genetically modified yeast further comprises exogenous nucleic acid coding for a carboxyreductase (CAR, EC1.2.99.6) and a phosphopantetheinyl transferase (PPTase). In a specific embodiment, the CAR enzyme is issued from the organism *Nocardia iowensis* or *Micobacterium smegmatis* and the PPTase is issued from *Nocardia iowensis* or *Micobacterium smegmatis.*

According to a specific embodiment of the invention, and as described in example 1, the artificial vanillin pathway is introduced into the locus bud31 of modified yeast cells. Nine synthetic DNA fragments are needed to introduce artificial vanillin pathway in yeast genome (Figure 4). All genes possess one promoter and one terminator. Gene promoters allow constitutive expression of proteins from the pathway. Fragment hybridizes and recombines together in the region of the entire ORF of each couple of gene. By that way, the whole pathway is assembled in the yeast cell, and then integrated into the chromosome. Tg 5' and Tg 3' correspond to the target sequences on the yeast genome that corresponds to the insertion site in the BUD 31 locus of the yeast chromosome triggering the homologous integration into the desired chromosome site. HIS3 and LEU2 are the flanking markers enabling the double selection of the recombinant pathway. After assembly of the fragments by homologous recombination in yeast, a functional complete pathway of 30Kbp is reconstituted and the double selection permits the isolation of recombinants.

In a particular embodiment of the invention, the genetically modified yeast further comprises exogenous nucleic acids coding for a vanillyl alcohol oxidase (EC 1.1.3.38) that catalyzes the chemical conversion of vanillyl alcohol and O₂ into vanillin and H₂O₂. Presence of this enzyme allows a better yield of vanillin since all vanillyl alcohol produced via the artificial biosynthetic pathway can be converted into vanillin. A specifically preferred vanillyl alcohol oxidase is the enzyme described in patent US 5,721,125.

### Enzymes involved in the biosynthesis pathway of vanilloids from dehydroshikimic acid

According to a second embodiment of the invention, the artificial vanillin pathway derives from the acid dehydroshikimic pathway, such as disclosed in Hansen *et al.,* 2009 (cited above). In particular, the genetically modified yeast comprises a set of exogenous nucleic acids coding for the following enzymes able to convert an acid dehydroshikimic into a vanilloid: a 3-dehydroshikimate dehydratase (3DSD), an aromatic carboxylic acid reductase (ACAR) and a catechol O-methyltransferase or a caffeic acid O-methyltransferase (both designed as COMT). Preferentially, the genetically modified yeast further comprises a phosphopantetheinyl transferase (PPTase).

In a specific aspect of the invention, and for all disclosed modified yeast, the exogenous nucleic acid encoding the caffeic acid O-methyltransferase (COMT) is preferentially issued from the plants *Medicago sativa, Vanilla planifolia* or *Rosa chinensis.* Interestingly, these enzymes have a high selectivity for methylation, and their final product is pure vanillin (100% of selectivity) instead of a mixture of vanillin and isovanillin.

### Recombinant yeasts

In the sense of the present invention, it is understood that exogenous nucleic acids encoding polypeptides that are introduced into the yeast are "codon-optimized" to be expressed efficiently into the yeast. The man skilled in the art knows some 'Codon Optimization Tools' that allows the preparation of synthetic genes from one host organism for an optimized expression in another, in particular in the yeast according to the invention.

Exogenous nucleic acids encoding the heterologous polypeptides are introduced into the yeast by transformation and this manipulation results into incorporation and expression of exogenous genetic material. Transformation of yeast can be performed by means well known by the man skilled in the art : yeast cells may be treated with enzymes to degrade their cell walls, may be exposed to alkali cations such as lithium acetate, or to polyethylene glycol. Electroporation using electric shock is also a technique that allows exogenous DNA to enter into yeasts. Some of useful techniques are reviewed in the review (Kawai S, Hashimoto W, Murata K. "Transformation of Saccharomyces cerevisiae and other fungi: methods and possible underlying mechanism. " Bioeng Bugs. 2010 Nov-Dec;1(6):395-403).

In a preferred embodiment of the invention, transformations of competent yeast cells are performed as described by Gietz and Woods (Gietz and Woods. "Transformation of yeast by the LiAc/ss Carrier DNA/PEG method. " Meth. Enzymol., 350, 87-96).

An equimolar mix of fragments is used to transform yeast.

According to a specific aspect of the invention, the polypeptides are encoded by exogenous genes stably integrated into the yeast genome.

Preferentially, the nucleic acids encoding heterologous polypeptides are integrated into the yeast genome by the technique of homologous recombination. An example of integration into genome of the yeast is shown in figure 4. Preferably, in a set of exogenous nucleic acids as described herein, the exogenous nucleic acids are assembled into a cluster and said cluster is integrated into the yeast genome. In particular, polynucleotides encoding a series of enzymes are provided as full-length polynucleotides of different origin, wherein :
- the 5'-terminal sequence is of the polynucleotide encoding the first enzyme in the series, and
- the 3'-terminal sequence is of the polynucleotide encoding the last enzyme in the series.

Most preferably, each nucleic acid encoding a polypeptide included in an exogenous nucleic acid as described herein comprises, from its 5'-end to its 3'-end, (1) a regulatory sequence comprising one or more promoter sequences, the said regulatory sequence being functional in the yeast organism wherein it has been introduced, (ii) an ORF encoding a polypeptide of interest and (iii) one or more transcription terminator sequences.

The polynucleotides encoding the enzymes may be in the order of the consecutive enzymatic reactions required for the biosynthesis pathway which is under consideration, or in any other order, provided that the required enzymes are actually produced.

The invention also relates to a recombinant yeast having a reduced alcohol dehydrogenase activity, in the genome of which has been inserted a DNA construct comprising :
- one or more nucleic acids encoding a phenylalanine ammonia lyase (PAL) or a tyrosine ammonia lyase (TAL),
- one or more nucleic acids encoding a cinnamate-4-hydrolase (C4H),
- one or more nucleic acids encoding a 4-coumarate coenzyme A ligase (4CL),
- one or more nucleic acids encoding a enoyl-CoA hydratase/aldolase (ECH),
- one or more nucleic acids encoding a hydroxybenzoic acid hydroxylase (HBH) and
- one or more nucleic acids encoding a caffeic acid O-methyltransferase (COMT),
and wherein in said recombinant yeast the decarboxylation of phenylacrylic acids is attenuated, in particular said yeast does not comprise a functional gene *fdc1* and/or *pad1.*

The invention also relates to a recombinant yeast such as described above, in the genome of which has been inserted a DNA construct comprising:
- one or more exogenous nucleic acids encoding a carboxyreductase (CAR) and
- one or more exogenous nucleic acids encoding a heterologous phosphopantetheinyl transferase (PPTase).

The invention also relates to a recombinant yeast having a reduced alcohol dehydrogenase activity, in the genome of which has been inserted a DNA construct comprising:
- one or more nucleic acids encoding a 3-dehydroshikimate dehydratase (3DSD),
- one or more nucleic acids encoding an aromatic carboxylic acid reductase (ACAR)
- one or more nucleic acids encoding a phosphopantetheinyl transferase (PPTase), and
- one or more nucleic acids encoding a caffeic acid O-methyltransferase (COMT),
and wherein said recombinant yeast does not comprise a functional gene *ald6.*

According to the invention, the yeast is from the genera *Saccharomyces or Schizosaccharomyces.* In particular the modified yeast belongs to the following species: *Saccharomyces cerevisiae Schizosaccharomyces pombe, Pichia pastoris, Yarrowia lipolytica.*

### Culture process for producing a vanilloid or a precursor thereof

The present invention is also related to a method for producing a vanilloid or a precursor thereof, comprising the following successive steps:
a) providing a recombinant yeast comprising enzymes necessary for vanilloid biosynthesis,
b) cultivating said yeast in an appropriate medium comprising a source of carbon chosen from glucose, fructose, galactose, arabinose, lactose, ethanol, glycerol, and mixtures thereof; and
c) isolating said vanilloid or precursor thereof from the medium.

The phrase "recombinant yeast comprising enzymes necessary for vanilloid biosynthesis" designates a genetically modified yeast, expressing enzymes allowing the biosynthesis of a vanilloid in said yeast by any biosynthesis pathway.

In particular, exogenous nucleic acids are inserted into said yeast, said nucleic acids coding for enzymes involved in the biosynthesis pathway of a vanilloid, being chosen among the following groups of enzymes:
- enzymes able to convert an aromatic amino acid into a vanilloid; or
- enzymes able to convert an acid dehydroshikimic into a vanilloid.

In particular, said recombinant yeast does not comprise a functional gene *adh6.* More particularly, said yeast does not comprise a functional gene *adh6* neither a functional gene *fdc1.* More particularly, said yeast does not comprise a functional gene *adh6* neither a functional gene *pad1.*

In a preferred aspect of the invention, said recombinant yeast does not comprise functional genes *adh6, pdc1, fdc1.*

In another preferred aspect of the invention, said recombinant yeast does not comprise functional genes *adh6, pad1, fdc1.*

In another preferred aspect of the invention, said recombinant yeast does not comprise functional genes *adh6, pad1, pdc1.*

In another preferred aspect of the invention, said recombinant yeast does not comprise functional genes *adh6, fdc1, pad1* and *aro10.*

In a specific aspect of the invention, at least one of the genetically modified yeasts described in the present application is cultivated in this method.

Terms 'biosynthesis', "bioconversion" 'fermentative production' and 'production' have the same meaning in the sense of the invention, and designates the production of at least one vanilloid by a recombinant yeast cultivated in appropriate conditions.

The term "precursor" as used herein refers to a substrate molecule that is subject to enzymatic reactions and can be converted into a product by this specific reaction. By "a precursor of vanilloid" is meant a precursor or an intermediate in the biosynthesis pathway of a vanilloid, which is in particular a hydroxybenzaldehyde or a respective acid or a respective alcohol, an aromatic amino acid, or an acid dehydroshikimic. This term includes in particular: protocatechuic aldehyde, 4-hydroxyaldehyde, protocacheuic alcohol, 4-hydroxybenzoic acid, 4-hydroxybenzyl alcohol, protocacheuic acid, protocacheuic alcohol, 4-hydroxyaldehyde, 4-hydroxyl alcohol, cinnamic acid, coumaric acid, caffeic acid and ferulic acid.

According to the invention the term 'cultivating' is used to denote the growth of yeast. The term "appropriate medium" designates a medium (e.g., a sterile, liquid media) comprising nutrients essential or beneficial to the maintenance and/or growth of the cell such as carbon sources or carbon substrates, nitrogen sources, urea, ammonium sulfate, ammonium chloride, ammonium nitrate and ammonium phosphate; phosphorus sources; metal salts, for example magnesium salts, cobalt salts and/or manganese salts; as well as growth factors such as amino acids and vitamins. Typical medium is composed of 10 g/l of yeast extract, 20 g/l of peptone and 20 g/l of carbon source.

The term "source of carbon" or "carbon substrate" according to the present invention denotes any source of carbon that can be used by those skilled in the art to support the normal growth of a yeast. Yeasts use sugars as their main carbon and energy source, but non-conventional carbon sources can be accepted too. The major source for energy production in the yeast is glucose and glycolysis is the general pathway for conversion of glucose to pyruvate, whereby production of energy in form of ATP is coupled to the generation of intermediates and reducing power in form of NADH for biosynthetic pathways. Fructose is also a hexose that can be uptake and metabolized by yeast. Galactose is a 'non-conventional' nutrient for yeast, which however can be used as a sole carbon source when glucose is absent from the medium. In yeast cells supplied with glucose, the GAL genes are repressed. They are activated a thousand fold in cells that are starved for glucose, and this one of the few pathways in yeast which is regulated in a nearly 'all-or-nothing' mode. Enzymes implied in conversion of galactose to glucose 1-phosphate are: galactose kinase, galactose-1-phosphate-urydiltransferase, and UDP-glucose-4-epimerase. Glycerol functions as a compatible solute in osmoregulation in osmotolerant yeasts that are capable of growing in high sugar or salt environments. Many types of yeast can grow on glycerol as a sole carbon source under aerobic conditions, but glycerol is a non-fermentable carbon source for many yeast species, including *S. cerevisiae.* To serve as a carbon source, glycerol after internalization has to convert by glycerol kinase to glycerol-3-phosphate, which is then transformed into dihydroxyacetone phosphate by glycerol-3-phosphate dehydrogenase that is a substrate in gluconeogenesis.

Many types of yeast have the capability of metabolizing ethanol or methanol. In presence of ethanol ADH2, the enzyme that converts ethanol back into acetaldehyde, is expressed. Acetaldehyde is subsequently converted into acetyl-CoA, the substrate for the citric acid cycle.

The source of carbon is selected among the group consisting of glucose, fructose, mannose, xylose, arabinose, galactose, lactose, ethanol, cellobiose, glycerol and polysaccharides such as cellulose, and mixtures thereof. An especially preferred substrate is ethanol.

According to another preferred embodiment of the invention, the volume of the culture is at least one liter. Indeed, when the culture volume is low (about 1 ml), vanillin is produced but is quickly converted to vanillic acid. This conversion is not observed in stable continuous culture in larger volume, of at least one liter.

The cultivation process comprises a fermentation process (batch, fed-batch or continuous mode) under controlled conditions of pH, temperature, pO₂ and aeration, conditions well known to a person skilled in the art.

In a specific embodiment, the vanilloid isolated from the medium is selected from the group consisting of: vanillin, vanillic acid, vanillyl alcohol and vanillin-glycoside.

In another specific embodiment, the vanilloid precursor isolated from the medium is selected from the group consisting of protocatechuic aldehyde, protocacheuic acid, protocacheuic alcohol, 4-hydroxyaldehyde, 4-hydrobenzoic acid, 4-hydroxyl alcohol, cinnamic acid, coumaric acid, caffeic acid and ferulic acid.

According to a specific embodiment, the present invention is related to a method for producing a vanilloid comprising the following successive steps:
a) providing a recombinant yeast comprising enzymes necessary for vanilloid biosynthesis,
b) cultivating said yeast in an appropriate medium comprising a source of carbon chosen from glucose, fructose, galactose, arabinose, lactose, ethanol, glycerol, and mixtures thereof;
c) eventually, isolating said vanilloid from the medium;
   and further comprising a step d:
d) converting precursors of vanilloid remaining in the medium into vanilloid.

### Culture process using bi-phase culture

Vanillin cytotoxicity is a bottleneck in the microbial bioconversion including yeasts. We proceeded to an extractive bioconversion of vanillin using polypropylene glycol. In the two-phase process, the bioconversion is performed in a medium comprising an aqueous phase containing the biocatalyst (cells) and hydrophobic organic solvent (water-immiscible phase) containing organic compounds as substrates and / or products. The organic solvent acts as a reservoir for hydrophobic compounds. The biphasic system enables continuous supply of substrate depending on the metabolic demand and without inhibiting the enzymatic reaction. Moreover, the product of the reaction is progressively extracted into the organic phase. A major advantage is to reduce the cytotoxicity of substrates and/or products of the bioconversion. This system allows raising yields of bioconversion.

PPG is a polymer of propylene glycol also called propane-1,2-diol or 1,2-propanediol. When aqueous solutions (culture medium) and PPG are mixed, the mixture becomes turbid and when it is left for a while, phase separation occurs. Aqueous two-phase system therefore provides mild conditions for cells and proteins. The interfacial tension is very low compared with that of organic solvent /water system. The phase system forms a finely dispersed emulsion with large surface area and short diffusion distances from one phase to the other. This means that conditions are very favorable for mass transfer.

The present invention is also related to a method for producing a vanilloid or a precursor thereof, comprising:
a) providing a recombinant yeast comprising enzymes necessary for vanilloid biosynthesis,
b) cultivating said yeast in an appropriate medium comprising a precursor chosen from glucose, fructose, galactose, arabinose, lactose, ethanol, glycerol, and mixtures thereof; and
c) isolating said vanilloid or precursor thereof from the medium,
wherein the step b) is performed in an appropriate medium comprising two phases, an aqueous phase and a water immiscible phase, said water immiscible phase preferably consisting of polymer of propylene glycol, fatty alcohol, fatty acid, fatty ester or an oil.

The appropriate medium comprises precursors of the step b) or mixtures of thereof, either in pure grade or technical grade.

The cultivation process comprises a fermentation process (batch, fed-batch or continuous mode) under controlled conditions of pH, temperature, pO₂ and aeration, conditions well known to a person skilled in the art.

The phrase "recombinant yeast comprising enzymes necessary for vanilloid biosynthesis" designates a genetically modified yeast, expressing enzymes allowing the biosynthesis of a vanilloid in said yeast by any biosynthesis pathway.

In particular, exogenous nucleic acids are inserted into said yeast, said nucleic acids coding for enzymes involved in the biosynthesis pathway of a vanilloid, being chosen among the following groups of enzymes:
- enzymes able to convert an aromatic amino acid into a vanilloid; or
- enzymes able to convert an acid dehydroshikimic into a vanilloid.

In particular, said recombinant yeast does not comprise a functional gene *adh6.* More particularly, said yeast does not comprise a functional gene *adh6* neither a functional gene *fdcl.* More particularly, said yeast does not comprise a functional gene *adh6* neither a functional gene *fdc1.* More particularly, said yeast does not comprise a functional gene *adh6* neither functional genes *fdc1* nor *pad1.*

In another preferred aspect of the invention, said recombinant yeast does not comprise functional genes *adh6, pad1, pdc1.*

In another preferred aspect of the invention, said recombinant yeast does not comprise functional genes *adh6, fdc1, pad1* and *aro10.*

In a specific aspect of the invention, the genetically modified yeasts described in the present application are cultivated according to this method, in a bi-phase medium, wherein said medium comprises two phases, an aqueous phase and a water immiscible phase, said water immiscible phase preferably consisting of: polymer of propylene glycol, fatty alcohol, fatty acid, fatty ester or an oil.

### Composition comprising vanilloid or a precursor thereof

The present invention is also related to a composition comprising a vanilloid or a precursor thereof obtainable by the method as disclosed above, and to the use of said composition as a flavoring in the human and animal nutrition field, in pharmacy, and as a fragrance in the cosmetics, perfumery and detergency industries.

Vanillin is a compound well-known for its aromatic properties. However, the flavor profile of an aromatic composition is dependent on for instance byproducts and impurities, and consequently on the preparation process. The composition comprising a vanilloid or a precursor thereof obtainable by the method of the invention may thus show aromatic notes different from other aromatic composition.

### EXAMPLES

### Example 1. Introduction of a vanillin biosynthesis pathway into Δadh6 yeast strain

All of the *Saccharomyces cerevisiae* strains used in this work were isogenic haploids from BY4741 and were obtained from EUROSCARF (haploid a-mater BY00 or α-mater BY10). Yeast strain BY47 derived from a strain collection that contains knock outs of auxotrophic (-ura3, -leu2, -his3) marker genes. Enrichment and propagation of clones were made in YPD liquid cultures (10 g/l bacto-yeast extract, 20 g/l bacto-peptone and 2% dextrose) at 30°C. Recombinants were selected on dropout agar plates (YNB + CSM) in the absence of uracil, leucine or histidine. The gene defects in uracil, histidine and leucine biosynthetic pathway result in auxotrophy. All ORF used for the pathway were synthesized by GeneArt (Germany) and then amplified by PCR. Amplification was performed using high fidelity PhusionTaq (New England Biolabs). Amplicons were cleaned up by using the Wizard PCR Clean-up System (Promega) and used for transformation assays.

The following genes were used:

**Table 1: Reference identities of the genes/proteins used in this example**

| Protein | *Species* | Reference NCBI | Length in amino acids | SEQ ID |
|---|---|---|---|---|
| PAL | *Petroselinum crispum* | X81158.1 GI:534892 | 718 AA | NO. 2 |
| | *Populus trichocarpa x Populus deltoides* | L11747.1 GI:169453 | 715 AA | NO. 1 |
| C4H | *Glycine max* | FJ770468.1 GI:225194700 | 506 AA | NO. 3 |
| | *Petroselinum crispum* | Q43033.1 GI:3915088 | 506 AA | NO. 4 |
| 4CL | *Populus tremuloides* | AF041049.1 GI:3258634 | 570 AA | NO. 5 |
| ECH | *Pseudomonas fluorescens* | AJ536325.1 | 276 AA | NO. 6 |
| | *Azotobacter vinelandii* | YP 002798614.1 GI:226943541 | 276 AA | NO. 7 |
| HBH | *Pseudomonas aeruginosa* | ZP_07797957.1 GI:313112178 | 394 AA | NO. 8 |
| | *Azotobacter vinelandii* | NC 012560.1 GI:226943557 | 394 AA | NO. 9 |
| COMT | *Medicago sativa* | ACYO6328.1 GI:261889456 | 365 AA | NO. 10 |
| | *Vanilla planifolia* | AAS64572.1 G1:45444737 | 365 AA | NO. 11 |
| CAR | *Nocardia iowensis* | AAR91681.1 GI:40796035 | 1174 AA | NO. 12 |
| PPTase | *Nocardia iowensis* | AB183656.1 GI:114848891 | 222 AA | NO. 13 |

The complete pathway was assembled using a somatic *in vivo* DNA assembly. Eight fragments containing (F1 to F8) the 6 genes of the vanillin pathway were designed by computational analysis. Fragments hybridize and recombine together in the region of the entire ORF of each couple of gene. That way enabled the whole pathway assembly in the yeast cell, and its integration into the chromosome. T 5' and T 3' correspond to the bud31 target sequences on the yeast genome allowing homologous integration onto the chromosome locus. Overlapping sequences correspond to the 5' part and the 3' part of the marker genes. HIS6 and LEU2 are the flanking markers enabling the double selection of the recombinant pathway. Each gene is under the control of one promoter and one terminator sequences allowing its expression in yeast cells. After assembly of the fragments by homologous recombination in yeast, a functional complete pathway is reconstituted and the double selection permits the isolation of recombinants.

Transformations of competent yeast cells were performed as described by Gietz and Woods (Transformation of yeast by the LiAc/ss Carrier DNA/PEG method. Meth. Enzymol., 350, 87-96). Analysis of clones revealed that the genes had assembled, resulting in correct ORFs.

HPLC was used to measure the production of vanillin and pathway intermediates in *S. cerevisiae* cultures. Analysis showed that chromatograms from cells expressing vanillin pathway genes contained additional peaks compared to a yeast control. These peaks were identified as cinnamic acid, coumaric acid, 4-hydroxybenzoic acid, 3,4-dihydroxybenzoic acid, vanillic acid and vanillin were identified. When the culture is not supplemented by exogenous phenylalanine, the PAL protein uses endogenous phenylalanine. The pathway is fully functional as 3,4-dihydroxybenzoic acid and vanillic acid were detected but the yield is reduced 4 times when compared to the phenylalanine supplemented medium. Only 1µM of vanillin was detected in the non-adapted strain.

A carboxylic acid reductase protein (CAR) was added with its activating coupling protein phosphopantetheinyl transferase (PPTase). The bicistronic construction was integrated to yeast genome by homologous recombination using URA as selection marker into ADH6 locus. Prevention of reduction of vanillin to vanillyl alcohol was achieved by knockout of the host alcohol dehydrogenase ADH6. In order to take off selection marker, flanking repeated sequences were added to URA3 gene in order to permit URA3 gene excision. Recombinant cells were selected on -URA selective medium and the right integration was verified by PCR.

### Example 2-Improvement of aldehyde stability in S. cerevisiae

Vanillin biosynthesis pathway used in our production process implies three aldehydes including the final product vanillin. One way to increase vanillin production yield during a bioproduction using *S. cerevisiae* is to reduce aldehyde oxidase or aldehyde reductase endogenous activity in the production strain. A large number of Euroscarf deletion strains have been screened; these strains were firstly selected on the basis of a biochemical study and rational targeting of potentially interesting genes such as ADH (alcohol dehydrogenases), ALD (aldehyde dehydrogenases), GRE (aldehyde reductases), OYE, AAD (aryl alcohol dehydrogenase) & ARI (aldehyde reductase).

**Table 2: Euroscarf deletion strains**

| **Gene Name** | **Description** | **Systematic Name** | **Alias(es)** | **Euroscarf Acc N°** |
|---|---|---|---|---|
| ADH5 | Alcohol dehydrogenase iso enzyme V | YBR145W | alcohol dehydrogenase ADH5 | Y03284 |
| ADH7 | NADPH-dependent medium chain alcohol dehydrogenase | YCR105W | ADHVII | Y05821 |
| SFA1 | Bifunctional alcohol dehydrogenase and formaldehyde dehydrogenase | YDL168W | ADH5, bifunctional alcohol dehydrogenase/S-(hydroxymethyl)glutathione dehydrogenase | Y03866 |
| ADH4 | Alcohol dehydrogenase iso enzyme type IV | YGL256W | NRC465, ZRG5, alcohol dehydrogenase ADH4 | Y04623 |
| ADH3 | Mitochondrial alcohol dehydrogenase isozyme III | YMR083W | alcohol dehydrogenase ADH3 | Y06217 |
| ADH2 | Glucose-repressible alcohol dehydrogenase II | YMR303C | ADR2, alcohol dehydrogenase ADH2 | Y00891 |
| ADH 6 | NADPH-dependent medium chain alcohol dehydrogenase | YMR318C | ADHVI | Y06460 |
| ALD5 | Mitochondrial aldehyde dehydrogenase | YER073W | aldehyde dehydrogenase (NAD(P)(+)) ALD5 | Y00213 |
| ALD3 | Cytoplasmic aldehyde dehydrogenase | YMR169C | aldehyde dehydrogenase (NAD(+)) ALD3 | Y00752 |
| ALD2 | Cytoplasmic aldehyde dehydrogenase | YMR170C | aldehyde dehydrogenase (NAD(+)) ALD2 | Y00753 |
| ALD4 | Mitochondrial aldehyde dehydrogenase | YOR374W | ALD7, ALDH2, aldehyde dehydrogenase (NADP(+)) ALD4 | Y01661 |
| ALD 6 | Cytosolic aldehyde dehydrogenase | YPL061W | ALD1, aldehyde dehydrogenase (NADP(+)) ALD6 | Y02767 |
| GRE3 | Aldose reductase | YHR104W | trifunctional aldehyde reductase/xylose reductase/glucose 1-dehydrogenase (NADP(+)) | Y01932 |
| GRE2 | 3-methylbutanal reductase and NADPH-dependent methylglyoxal reductase | YOL151W | methylglyoxal reductase (NADPH-dependent) GRE2 | Y07320 |
| OYE2 | Conserved NADPH oxidoreductase containing flavin mononucleotide (FMN) | YHR179W | Old Yellow Enzyme | Y02873 |
| OYE3 | Conserved NADPH oxidoreductase containing flavin mononucleotide (FMN) | YPL171C | ZRG6, Old Yellow Enzyme | Y02081 |
| AAD3 | Putative aryl-alcohol dehydrogenase | YCR107W | | Y07253 |
| AAD4 | Putative aryl-alcohol dehydrogenase | YDL243C | | Y03940 |
| AAD 6 | Putative aryl-alcohol dehydrogenase | YFL056C | | Y05677 |
| AAD10 | Putative aryl-alcohol dehydrogenase | YJR155W | | 10327A |
| AAD14 | Putative aryl-alcohol dehydrogenase | YNL331C | | 10565A |
| ARI1 | NADPH-dependent aldehyde reductase | YGL157W | carbonyl reductase (NADPH-dependent) ARI1 | Y04524 |

A screening has been performed to evaluate stability of the three aldehydes of the vanilloid pathway with these 22 strains and results have been compared with the same approach with a wild-type *S. cerevisiae* strain. Principle of the assay is to grow yeasts for a first 24h in rich-medium in a 96-well microplates. Then cells are two fold diluted using the same quantity of medium and let grown 24h more in presence of 500µM of the aldehyde to be evaluated (4-hydroxybenzaldehyde, 3,4-dihydroxybenzaldehyde and vanillin).

Figure 3 shows the titers obtained for three oxidative states (aldehyde, acid, alcohol) related to the feeding.

Looking at these results, the strongest impact is given by *adh6* deletion. ALD6 deletion also has an impact on the quantity of acid formed (more acid than the wild type cells). ALD6 could work on the conversion of aldehyde to alcohol. The third strong effect concerns ADH3. Actually, alcohol concentration is higher in Δ*adh3* cells than in the WT. ADH3 probably catalyzes the conversion of aldehyde into its acid form. A very small effect has been detected for Δ*gre3* and Δ*ald2.* These genes were then assayed in combination with the most promising ones. Looking at all these results, the goal was then to combine gene deletions in the same cell. An expression of the van pathway in such a genetic context could favor vanillin synthesis.

Figures 3 show that the major effect is given by ADH6 deletion in WT cells, as expected. However a little effect is also detected when ADH3 deletion is added to that of ADH6. Indeed, alcohol concentration is a little lower in this strain.

Δ*ald6* is another deletion that significantly disturbs the equilibrium between the three oxidation states of 4HB and 3,4diHB. Since it doesn't show a strong impact on vanillin, we did not try it during the first assay of multi-deletion. However, the low impact of the other double deletions convinced us to try a deletion of *ald6* in combination with Δ*adh6.*

### Example 3. Effect of metabolic engineering on vanillin pathway flux - Multideletion of pad1, fdc1 & aro10 genes

The deletion of three genes coding for enzymes involved in the catabolism of phenylpropanoid compounds *(fdc1, pad1* & *aro10)* were tested. Each new gene deletion and combinations thereof were evaluated for the production of vanillin and intermediates of the vanillin pathway. Taking into account the already obtained results in the investigation of metabolic engineering, the tested gene deletions were: *aro10, fdc1, pad1, adh6, ald6, adh3.*

*Aro10* deletion increases the intrinsic concentration of phenylalanine, which is the branching point of our pathway. *Fdc1* and *pad1* deletions prevent the decarboxylation of intermediate metabolites, such as cinnamic and coumaric acids.

Gene deletion is performed using a CreLox deletion system. First, a deletion cassette is obtained by PCR on an appropriate plasmid (pUG72 or pUG73 from Euroscarf). The forward Primers used for amplification of the deletion K7 are designed to add upstream of the 5' side of the selection marker a track of 50 bp homologous to the last 50 bp of the promoter of the gene to be deleted. The reverse ones are designed to add downstream of the 3' side of the selection marker a track of 50 bp homologous to the first 50 bp of the terminator of the gene to be deleted. Once transformed by an appropriate deletion K7, the selection marker *(Ura3* or *Leu2)* is integrated in the yeast genome and it strictly replaces the ORF of the targeted gene. The selection marker is framed by two identical sequences (LoxP) that allow marker excision under the action of a Cre enzyme obtained by a new transformation of the new strain using a pSH62 plasmid (Euroscarf). *His3* selection marker of the pSH62 is then removed by a little growth of the transformed cells in a rich medium. This deletion system has been used to successively delete genes and to retrieve selection markers for next transformations. Each new gene deletion is followed by evaluation of cinnamic and coumaric acids production capacities after a transformation of the cells by a ZmPAL expression cassette.

Several modified yeast strains were evaluated for vanillin production; results are shown in figure 5:
Y10
Y10Δ*adh6* ;
Y10Δ*adh6*Δ*pad1*Δ*dc1;*
*Y10*Δ*aro10Δadh6Δfdc1;*
*Y10*Δ*aro10*Δ*adh6*Δ*fdc1*Δ*adh3*;
Y10*Δaro10*Δ*adh6Δfdc1*Δ*ald6*;
*Y10*Δ*aro10Δadh6Δfdc1Δadh3Δald6*;

Recombinant modified clones containing vanillin pathway were cultured in rich medium with galactose as carbon source using 100ml Erlenmeyer. After three days, clarified supernatants were analyzed by HPLC and concentration of metabolites form the pathway was evaluated. Total concentration of metabolites from vanillin pathway was evaluated.

Figure 5 shows the impact of deletions on the flow of the vanillin pathway. The total metabolites concentration (grey bars) comprises cinnamic acid, coumaric acid, 4-hydroxybenzaldehyde, 4-hydroxybenzoic acid, 3,4-dihydroxybenzoic acid, 3,4-dihydroxybenzaldehyde, vanillin, vanillic acid and vanillyl alcohol concentrations. Amount of final products (vanillin, vanillyl alcohol and vanillic acid) are indicated with striped bars and vanillin concentration is indicated with black bars.

*Aro10* deletion, by increasing the concentration of phenylalanine produced by the yeast, clearly increases the overall flow of the vanillin pathway. The total metabolite concentration is multiplied 4-fold. When the deletion of *ald6* is added, flow is also increased. In contrast, the *adh3* deletion decreases the flow of the pathway. Vanillin produced equilibrates into acid and alcohol oxidation states. The results show that *aro10* deletion has little effect on the final concentration of metabolites (vanillin, vanillic acid & vanillyl alcohol). Besides increasing the flow of the pathway, *ald6* deletion significantly increases the concentration of end metabolites.

Figure 6 shows the profile of supernatants of strains presenting the following deletions: Δadh6Δaro10Δfdc1 and Δadh6Δaro10Δfdc1Δpdc1. Clearly, the deletion of *pad1* is beneficial for the vanillin biosynthesis pathway in these strains.

### Example 4. Optimization of culture conditions to increase vanillin production - Yeast volume culture

The synthesis of vanillin was evaluated based on the volume of culture for bioconversion. From a pre-culture, YPD medium was inoculated to an OD₆₀₀ of 0.1. The cells were then distributed into the different kind of media support from 96-well plate to a 1 liter Erlenmeyer flask. The concentration of vanillin was found to be clearly dependent on the volume of the support. Culture in 96-well plate allows good growth of yeast, but very little metabolite pathway vanillin are produced. Using 24-well plates, vanillin is produced, but quickly converted to vanillic acid. In contrast, when the bioconversion takes place in 1 liter Erlenmeyer flask, the synthesis of vanillin is continuous and stable and reaches 250 µM after 8 days, and then the concentration drops down (Figure 7).

### Example 5. Extractive bioconversion of vanillin using polypropylene glycol (PPG)

The toxicity of vanillin for yeast was evaluated (Figure 8). A yeast culture of *Δadh6Δdc1Δpad1* strain expressing vanillin pathway was inoculated in YPDE to an OD₆₀₀ of 0.05 and yeast growth was monitored in the presence of different concentrations of vanillin (0 to 30 mM). The same experiment was carried out by adding 30% PPG 1200. Figure 7 shows that the toxicity threshold is multiplied by a factor 10.

In a second step we evaluated the bioconversion by our pathway in the presence of PPG. To do this, from a preculture in YPGal medium, an optimized cell expressing the pathway of vanillin was inoculated into 50 ml YPAGal in a 250 ml Erlenmeyer containing 50% of PPG. A control was done without PPG. The concentration of each metabolite was monitored in the control YPGal alone. Typically clarified supernatant was removed and analyzed by HPLC. In the biphasic culture, two phases were studied. Phase containing the supernatant was analyzed. PPG phase was taken and diluted to a volume of acetonitrile to be analyzed by HPLC.

In control culture, total metabolite concentration reaches 710 µM whereas in biphasic culture concentration reaches 2,800 µM, 75% (2,100 µM) were located in PPG phase and 25% (700 µM) were located in aqueous phase. As to compare to control culture, bioconversion yield is clearly improved when PPG is present and vanillin concentration increased from 100 µM to 735 µM (Figure 9). All intermediate metabolites of the pathway are also extractable in PPG, but the emulsion is sufficiently high to allow the availability of substrates to the cell while reducing any toxicity. This leads to a significant increase in the flux of the pathway (4 fold increase). Vanillin concentration was significantly increased (8 fold increase).

### Example 6. Optimization of carbon source precursor for yeast growth on vanillin factory cell

Different carbon sources have been tested as substrates to produce vanillin in recombinant modified cells: sugars such as hexoses (glucose, fructose, galactose) as well as alcohol compounds with two carbons (ethanol) or three carbons (glycerol).

Enrichment and propagation of clones were classically made in YPD liquid cultures (10 g/l bacto-yeast extract, 20 g/l bacto-peptone and 2% dextrose) at 30°C.

Several media were used: YPGal (10 g/l bacto-yeast extract, 20 g/l bacto-peptone and 3% galactose); YPF (10 g/l bacto-yeast extract, 20 g/l bacto-peptone and 2% fructose); YPDE (10 g/l bacto-yeast extract, 20 g/l bacto-peptone, 2% dextrose and 2% ethanol); YPGE (10 g/l bacto-yeast extract, 20 g/l bacto-peptone, 2% glycerol and 2% ethanol); YPG (10 g/l bacto-yeast extract, 20 g/l bacto-peptone, 2% glycerol); YPE (10 g/l bacto-yeast extract, 20 g/l bacto-peptone and 2% ethanol).

Vanillin producing cells were precultured in YPD or YPGal medium in a 10 mL for 24h, and used to inoculate 50mL appropriate medium in a 250 mL Erlenmeyer flask. Metabolites concentrations were monitored.

Figure 10 shows that when yeasts grow on glucose carbon source, vanillin concentration reaches 100 µM over 3 days, then concentration decreases in favor of vanillic acid. Results are similar when yeasts grow on galactose carbon source, but vanillin is more stable. Fructose carbon source gives concentration of vanillin smaller than both other sugars. However, YPDE and YPGE give same results, vanillin concentration grows. According to concentration of vanillin when yeasts grow on glyceraldehyde carbon source as to compare to ethanol alone, ethanol was the key factor to increase vanillin concentration. Ethanol is a normal by-product of glucose fermentation, transiently accumulated in the yeast culture medium. But when it is added to medium in high concentration (2% or more), it influences yeast metabolism. Besides being a source of carbon for yeast growth, ethanol influences integrity of cellular membrane and carbon metabolism *of S. cerevisiae.* Addition of ethanol significantly changes the permeability of the cytoplasmic membrane, and results in a marked decrease in the mitochondrial membrane potential. In the present case, ethanol significantly increases vanillin synthesis.

### Conclusion

The synthesis of vanillin was significantly enhanced by the use of the process as described. Gene deletions have increased the flow of pathway by promoting the synthesis process and reducing the degradation process. The control of culture parameters has allowed the increase of the bioconversion. These combined changes have allowed the increase of the concentration of the recovered vanillin from 1 to 250 µM.

### SEQUENCES

PAL pop_715AA (SEQ ID 1)
METVTKNGYQNGSLESLCVNQRDPLSWGVAAEAMKGSHLDEVKRMVADYRKPVVKLGGET
LTIAQVASIAGHDTGDVKVELSESARPGVKASSDWVMDSMDKGTDSYGVTTGFGATSHRR
TKQGGALQKELIRFLNAGIFGNGTETCHTLPHSATRAAMLVRINTLLQGYSGIRFEILEA
ITRLLNNNITPCLPLRGTITASGDLVPLSYIAGLLTGRPNSKATGPTGEVLDAAEAFKAA
GIESGFFELQPKEGLALVNGTAVGSGLASMVLFETNVLAVLSELLSAIFAEVMNGKPEFT
DHLTHKLKHHPGQIEAAAIMEHILDGSAYMKAAKKLHETDPLQKPKQDRYALRTSPQWLG
PQIEVIRFSTKSIEREINSVNDNPLIDVSRNKAIHGGNFQGTPIGVSMDNVRLAIASIGK
LLFAQFSELVNDFYNNGLPSNLTASRNPSLDYGFKGAEIAMASYCSELQYLANPVTTHVQ
SAEQHNQDVNSLGLISSRKTAEAVDILKLMSTTFLVALCQAIDLRHLEENLKSAVKNTVS
QVSKRVLTTGANGELHPSRFCEKELLKVVDREYVFAYVDDPCSATYPLMQKLRQVFVDHA
LENGENEKNFSTSVFQKIEAFEEELKALLPKEVESARAAYDSGNSAIDNKIKECRSYPLY
KFVREELGTVLLTGEKVQSPGEEFDKVFTAMCQGKIIDPMLECLGEWNGSPLPIC

PALpet_718AA (SEQ ID 2)
MAYVNGTTNGHANGNGLDLCMKKEDPLNWGVAAEALTGSHLDEVKRMVAEYRKPVVKLEG
ETLTISQVAAISARDDSGVKVELSEEARAGVKASSDWVMDSMNKGTDSYGVTTGFGATSH
RRTKQGGALQKELIRFLNAGIFGSGAEAGNNTLPHSATRAAMLVRINTLLQGYSGIRFEI
LEAITKFLNHNITPCLPLRGTITASGDLVPLSYIAGLLTGRPNSKAVGPTGVTLSPEEAF
KLAGVEGGFFELQPKEGLALVNGTAVGSGMASMVLFEANILAVLAEVMSAIFAEVMQGKP
EFTDHLTHKLKHHPGQIEAAAIMEHILDGSAYVKAAQKLHEMDPLQKPKQDRYALRTSPQ
WLGPQIEVIRSSTKMIEREINSVNDNPLIDVSRNKAIHGGNFQGSPIGVSMDNTRLAIAA
IGKLMFAQFSELVNDFYNNGLPSNLSGGRNPSLDYGFKGAEIAMASYCSELQFLANPVTN
HVQSAEQHNQDVNSLGLISSRKTSEAVEILKLMSTTFLVGLCQAIDLRHLEENLKSTVKN
TVSQVAKRVLTMGVNGELHPSRFCEKDLLRVVDREYIFAYIDDPCSATYPLMQKLRETLV
EHALNNGDKERNLSTSIFQKIAAFEDELKALLPKEVETARAALESGNPAIPNRIKECRSY
PLYKFVREELGTEYLTGEKVRSPGEEFEKVFTAMSKGEIIDPLLECLESWNGAPLPIC

C4Hgly_506AA (SEQ ID 3)
MDLLLLEKTLIGLFLAAVVAIAVSTLRGRKFKLPPGPLPVPIFGNWLQVGDDLNHRNLTD
LAKKFGDIFLLRMGQRNLVVVSSPELAKEVLHTQGVEFGSRTRNVVFDIFTGKGQDMVFT
VYGEHWRKMRRIMTVPFFTNKVVQQYRHGWESEAAAVVEDVKKNPDAAVSGTVIRRRLQL
MMYNNMYRIMFDRRFESEEDPIFQRLRALNGERSRLAQSFEYNYGDFIPILRPFLKGYLK
ICKEVKETRLKLFKDYFVDERKKLGSTKSTNNNNELKCAIDHILDAQRKGEINEDNVLYI
VENINVAAIETTLWSIEWGIAELVNHPEIQQKLRDEIDRVLGAGHQVTEPDIQKLPYLQA
VVKETLRLRMAIPLLVPHMNLHDAKLGGYDIPAESKILVNAWWLANNPAHWKKPEEFRPE
RFFEEESLVEANGNDFRYLPFGVGRRSCPGIILALPILGITLGRLVQNFELLPPPGQSQI
DTSEKGGQFSLHILKHSTIVAKPRSF

C4Hpet_506AA (SEQ ID 4)
MMDFVLLEKALLGLFIATIVAITISKLRGKKLKLPPGPIPVPVFGNWLQVGDDLNQRNLV
DYAKKFGDLFMLRMGQRNLVVVSSPELAKDVLHTQGVEFGSRTRNVVFDIFTGKGQDMVF
TVYSEHWRKMRRIMTVPFFTNKVVQQYRFGWEDEAARVVEDVKANPEAATNGIVLRNRLQ
LLMYNNMYRIMFDRRFESVDDPLFLKLKALNGERSRLAQSFEYHFGDFIPILRPFLRGYL
KLCQEIKDKRLKLFKDYFVDERKKLESIKSVDNNSLKCAIDHIIEAQQKGEINEDNVLYI
VENINVAAIETTLWSIEWGIAELVNNPEIQKKLRHELDTVLGAGVQICEPDVQKLPYLQA

VIKETLRYRMAIPLLVPHMNLHDAKLAGYDIPAESKILVNAWWLANNPAHWNKPDEFRPE
RFLEEESKVEANGNDFKYIPFGVGRRSCPGIILALPILGIVIGRLVQNFELLPPPGQSKI
DTAEKGGQFSLQILKHSTIVCKPRSL

4CLpop_570AA (SEQ ID 5)
MMSVATVEPPKPELSPPQNQNAPSSHETDHIFRSKLPDITISNDLPLHAYCFENLSDFSD
RPCLISGSTGKTYSFAETHLISRKVAAGLSNLGIKKGDVIMTLLQNCPEFVFSFIGASMI
GAVITTANPFYTQSEIFKQFSASRAKLIITQSQYVNKLGDSDCHENNQKPGEDFIVITID
DPPENCLHFNVLVEASESEMPTVSILPDDPVALPFSSGTTGLPKGVILTHKSLITSVAQQ
VDGEIPNLYLKQDDVVLCVLPLFHIFSLNSVLLCSLRAGSAVLLMQKFEIGSLLELIQKH
NVSVAAVVPPLVLALAKNPLEANFDLSSIRVVLSGAAPLGKELEDALRSRVPQAILGQGY
GMTEAGPVLSMCLAFSKQPFPTKSGSCGTVVRNAELKVIDPETGRSLGYNQPGEICIRGS
QIMKGYLNDAEATANTIDVEGWLHTGDIGYVDDDDEIFIVDRVKEIIKFKGFQVPPAELE
ALLVNHPSIADAAVVPQKDEVAGEVPVAFVVRSDDLDLSEEAVKEYIAKQVVFYKKLHKV
FFVHSIPKSASGKILRKDLRAKLATATTMS

ECHpfl_276AA (SEQ ID 6)
MSNYEGRWTTVKVEIEDGIAWVILNRPEKRNAMSPTLNREMIDVLETLEQDPAAGVLVLT
GAGEAWTAGMDLKEYFREVDAGPEILQEKIRREASQWQWKLLRMYAKPTIAMVNGWCFGG
GFSPLVACDLAICADEATFGLSEINWGIPPGNLVSKAMADTVGHRQSLYYIMTGKTFGGQ
KAAEMGLVNDSVPLARLREVTIELARNLLEKNPVVLRAAKHGFKRCRELTWEQNEDYLYA
KLDQSRLLDTEGGREQGMKQFLDDKSIKPGLQAYKR

ECHavi_276AA (SEQ ID 7)
MNKYEGRWKTVIVEIEGGIAWVTLNRPDKRNAMSPTLNREMRDVLETLEQDPAARVLVLT
GAGSAWTAGMDLKEYFREVDAGPEILQEKIRREACEWQWKLLRMYAKPTVAMVNGWCFGG
GFSPLVACDLAICADEATFGLSEINWGIPPGNLVSKAMADTVGHRQALYYIMTGKTFDGR
QAAEMGLVNQSVPLAQLRETVATLCQDLLDKNPVVLRAAKNGFKRCRELTWEQNEDYLYA
KLDQSRLLDEEGGREEGMRQFLDEKSIKPGLQAYKR

HBHpae_394AA (SEQ ID 8)
MKTQVAIIGAGPSGLLLGQLLHKAGIDNVILERQTPDYVLGRIRAGVLEQGMVDLLREAG
VDRRMARDGLVHEGVEIAFAGQRRRIDLKRLSGGKTVTVYGQTEVTRDLMEAREACGATT
VYQAAEVRLHDLQGERPYVTFERDGERLRLDCDYIAGCDGFHGISRQSIPAERLKVFERV
YPFGWLGLLADTPPVSHELIYANHPRGFALCSQRSATRSRYYVQVPLTEKVEDWSDERFW
TELKARLPAEVAEKLVTGPSLEKSIAPLRSFVVEPMQHGRLFLAGDAAHIVPPTGAKGLN
LAASDVSTLYRLLLKAYREGRGELLERYSAICLRRIWKAERFSWWMTSVLHRFPDTDAFS
QRIQQTELEYYLGSEAGLATIAENYVGLPYEEIE

HBHavi_394AA (SEQ ID 9)
MKTQVAIIGAGPSGLLLGQLLHKAGIDNVILERHSPDYVLGRIRAGVLEQGVVDLLREAG
VAERMDREGLVHEGIELACSGRRIRLDLKALSGGKTVMVYGQTEVTRDLMDARRASGAPI
VYEAQNVRLSGLKDGMPHVTYEKDGQTHRLDCDYIAGCDGFHGVSRQSIPAEALSHYERV
YPFGWLGLLSDTPPVHEELIYAHTDLGFVLCSQRSPTRSRYYLQVPLSDRVEDWSDERFW
NELKRRLPGDVANRLVTGPSLEKSIAPLRSYVVEPMQYGRLFLVGDAAHIVPPTGAKGLN
LAGSDVCYLYRILLKVYREGRTELLEKYSELALRRVWKGERFSWFMTNLLHDFEGSDAFD
RRMQLADRDYYLDSEAGRVTIAENYVGLPYEEIA

COMTmsa_365AA (SEQ ID 10)
MGSTGETQITPTHISDEEANLFAMQLASASVLPMILKSALELDLLEIIAKAGPGAQISPI
EIASQLPTTNPDAPVMLDRMLRLLACYNILTCSVRTQQDGKVQRLYGLATVAKYLVKNED
GVSISALNLMNQDKVLMESWYHLKDAVLDGGIPFNKAYGMTAFEYHGTDPRFNKVFNKGM
SDHSTITMKKILETYTGFEGLKSLVDVGGGTGAVINTIVSKYPTIKGINFDLPHVIEDAP
SYPGVEHVGGDMFVSIPKADAVFMKWICHDWSDEHCLKFLKNCYEALPDNGKVIVAECIL
PVAPDSSLATKGVVHIDVIMLAHNPGGKERTQKEFEDLAKGAGFQGFKVHCNAFNTYIME
FLKKV

COMTvpl_365AA (SEQ ID 11)
MATWVEHQQQNGSKDVDEEACMYAMQLSSMVVLPMTLRVAVELGILEQIQAGGPDSYLT
AEDLAARLGNSNPLAPVMIERILRLLTSYSILNFTDTVDGEGRTVRSYGAAHVCKYLTPN
QDGVSMAPLVLMNTDKVLMESWYHMKDAVTNGGIPFNLAYGMTAFEYHGKDLRFNKVFNE
GMKNNSIIITKKILERYKRFEDVNVLIDVGGGIGGTISMITAKYPHIHGINFDLPHVVSE
APPFQGVEHVGGNMFESVPIGDAIFIKWILHDWSDEHCLKLLRNCAKSLPDKGKVIVVEC
ILPDAPLVTPEAEGVFHLDMIMLAHNPGGKERTKKEFKELAMLSGFSNFKALFSYANVWV
MEFNK

Carboxylic acid reductase_1174AA (SEQ ID 12)
MAVDSPDERLQRRIAQLFAEDEQVKAARPLEAVSAAVSAPGMRLAQIAATVMAGYADRPA
AGQRAFELNTDDATGRTSLRLLPRFETITYRELWQRVGEVAAAWHHDPENPLRAGDFVAL
LGFTSIDYATLDLADIHLGAVTVPLQASAAVSQLIAILTETSPRLLASTPEHLDAAVECL
LAGTTPERLVVFDYHPEDDDQRAAFESARRRLADAGSLVIVETLDAVRARGRDLPAAPLF
VPDTDDDPLALLIYTSGSTGTPKGAMYTNRLAATMWQGNSMLQGNSQRVGINLNYMPMSH
IAGRISLFGVLARGGTAYFAAKSDMSTLFEDIGLVRPTEIFFVPRVCDMVFQRYQSELDR
RSVAGADLDTLDREVKADLRQNYLGGRFLVAVVGSAPLAAEMKTFMESVLDLPLHDGYGS
TEAGASVLLDNQIQRPPVLDYKLVDVPELGYFRTDRPHPRGELLLKAETTIPGYYKRPEV
TAEIFDEDGFYKTGDIVAELEHDRLVYVDRRNNVLKLSQGEFVTVAHLEAVFASSPLIRQ
IFIYGSSERSYLLAVIVPTDDALRGRDTATLKSALAESIQRIAKDANLQPYEIPRDFLIE
TEPFTIANGLLSGIAKLLRPNLKERYGAQLEQMYTDLATGQADELLALRREAADLPVLET
VSRAAKAMLGVASADMRPDAHFTDLGGDSLSALSFSNLLHEIFGVEVPVGVVVSPANELR
DLANYIEAERNSGAKRPTFTSVHGGGSEIRAADLTLDKFIDARTLAAADSIPHAPVPAQT
VLLTGANGYLGRFLCLEWLERLDKTGGTLICVVRGSDAAAARKRLDSAFDSGDPGLLEHY
QQLAARTLEVLAGDIGDPNLGLDDATWQRLAETVDLIVHPAALVNHVLPYTQLFGPNVVG
TAEIVRLAITARRKPVTYLSTVGVADQVDPAEYQEDSDVREMSAVRVVRESYANGYGNSK
WAGEVLLREAHDLCGLPVAVFRSDMILAHSRYAGQLNVQDVFTRLILSLVATGIAPYSFY
RTDADGNRQRAHYDGLPADFTAAAITALGIQATEGFRTYDVLNPYDDGISLDEFVDWLVE
SGHPIQRITDYSDWFHRFETAIRALPEKQRQASVLPLLDAYRNPCPAVRGAILPAKEFQA
AVQTAKIGPEQDIPHLSAPLIDKYVSDLELLQLL

PPTase_222AA (SEQ ID 13)
MIETILPAGVESAELLEYPEDLKAHPAEEHLIAKSVEKRRRDFIGARHCARLALAELGEP
PVAIGKGERGAPIWPRGVVGSLTHCDGYRAAAVAHKMRFRSIGIDAEPHATLPEGVLDSV
SLPPEREWLKTTDSALHLDRLLFCAKEATYKAWWPLTARWLGFEEAHITFEIEDGSADSG
NGTFHSELLVPGQTNDGGTPLLSFDGRWLIADGFILTAIAYA

## Claims

1. A recombinant yeast comprising a set of exogenous nucleic acids, with each nucleic acid encoding a polypeptide involved in the biosynthesis pathway of a vanilloid, said set of exogenous nucleic acids encoding a set of polypeptides selected in a group comprising:
• a set of enzymes allowing conversion of an aromatic amino acid into a vanilloid; and
• a set of enzymes allowing conversion of an dehydroshikimic acid into a vanilloid;
and wherein the said recombinant yeast is devoid of a functional gene *adh6* encoding for an alcohol dehydrogenase,
and wherein said recombinant yeast has a reduced ability to decarboxylate phenylacrylic acids, preferably wherein said yeast does not comprise a functional *fdc1* gene and/ or a functional *pad1* gene.

2. A recombinant yeast according to claim 1, wherein said yeast does not comprise a functional gene *aro10.*

3. A recombinant yeast according to claim 1 or 2, wherein said yeast does not comprise a functional gene *ald6.*

4. A recombinant yeast according to anyone of claims 1 to 3, wherein said yeast does not comprise a functional gene *pdc1.*

5. A recombinant yeast according to anyone of claims 1 to 4, wherein the aromatic acid is phenylalanine or tyrosine, and wherein it comprises a set of exogenous nucleic acids coding for the following enzymes: tyrosine/phenylalanine ammonia lyase (TAL/PAL), cinnamate-4-hydrolase (C4H), 4-coumarate coenzyme A ligase (4CL), enoyl-CoA hydratase/aldolase (ECH), a hydroxybenzoic acid hydroxylase (HBH) and a caffeic acid O-methyltransferase (COMT).

6. A recombinant yeast according to claim 5, wherein it further comprises exogenous nucleic acids coding for a carboxyreductase (CAR) and a phosphopantetheinyl transferase (PPTase).

7. A recombinant yeast according to anyone of claims 5 or 6, wherein it further comprises exogenous nucleic acids coding for a vanillyl alcohol oxidase.

8. A recombinant yeast according to anyone of claims 1 to 4, wherein it comprises a set of exogenous nucleic acids coding for the following enzymes able to convert an acid dehydroshikimic into a vanilloid: a 3-dehydroshikimate dehydratase (3DSD), an aromatic carboxylic acid reductase (ACAR), a phosphopantetheinyl transferase (PPTase) and a caffeic acid O-methyltransferase (COMT).

9. A recombinant yeast according to anyone of claims 5 to 8, wherein the exogenous nucleic acid coding for caffeic acid O-methyltransferase (COMT) is issued from *Medicago sativa, Vanilla planifolia or Rosa chinensis.*

10. A recombinant yeast according to anyone of claims 1 to 9, wherein it is from the genera *Saccharomyces or Schizosaccharomyces.*

11. A recombinant yeast according to anyone of claims 1 to 10, wherein the polypeptides are encoded by exogenous genes stably integrated into the yeast genome.

12. A method for producing a vanilloid or a precursor thereof, comprising:
a) providing a recombinant yeast according to anyone of claims 1 to 11,
b) cultivating said yeast in an appropriate medium comprising a substrate chosen from glucose, fructose, galactose, arabinose, lactose, ethanol, glycerol, and mixtures thereof; and
c) isolating said vanilloid or said precursor from the medium.

13. A method according to claim 12 wherein the substrate is ethanol.

14. A method according to anyone of claims 12 or 13 wherein the vanilloid is selected from the group consisting of:
vanillin, vanillic acid, vanillyl alcohol and vanillin-glycoside,
or wherein the precursor is selected from the group consisting of:
protocatechuic aldehyde, protocacheuic acid, protocacheuic alcohol, 4-hydroxyaldehyde, 4-hydrobenzoic acid, 4-hydroxyl alcohol, cinnamic acid,
coumaric acid, caffeic acid and ferulic acid.

15. A method according to anyone of claims 12 to 14 wherein the step b) is performed in an appropriate medium comprising two phases, an aqueous phase and a water immiscible phase, said water immiscible phase preferably consisting of polymer of propylene glycol, fatty alcohol, fatty acid, fatty ester or an oil.

16. A composition comprising a vanilloid or a precursor thereof obtainable by the method as defined in anyone of claims 12 to 15.

17. Use of the composition according to claim 16 as a flavoring in the human and animal nutrition field, in pharmacy, and as a fragrance in the cosmetics, perfumery and detergency industries.


SEQUENCE LISTING

<110> RHODIA OPERATIONS

<120> Improved production of vanilloids by fermentation

<130> X20567

<160> 13

<170> BiSSAP 1.2

<210> 1
<211> 715
<212> PRT
<213> Populus trichocarpa x Populus deltoides


<400> 1
Met Glu Thr Val Thr Lys Asn Gly Tyr Gln Asn Gly Ser Leu Glu Ser
1               5                   10                  15
Leu Cys Val Asn Gln Arg Asp Pro Leu Ser Trp Gly Val Ala Ala Glu
            20                  25                  30
Ala Met Lys Gly Ser His Leu Asp Glu Val Lys Arg Met Val Ala Asp
        35                  40                  45
Tyr Arg Lys Pro Val Val Lys Leu Gly Gly Glu Thr Leu Thr Ile Ala
    50                  55                  60
Gln Val Ala Ser Ile Ala Gly His Asp Thr Gly Asp Val Lys Val Glu
65                  70                  75                  80
Leu Ser Glu Ser Ala Arg Pro Gly Val Lys Ala Ser Ser Asp Trp Val
                85                  90                  95
Met Asp Ser Met Asp Lys Gly Thr Asp Ser Tyr Gly Val Thr Thr Gly
            100                 105                 110
Phe Gly Ala Thr Ser His Arg Arg Thr Lys Gln Gly Gly Ala Leu Gln
        115                 120                 125
Lys Glu Leu Ile Arg Phe Leu Asn Ala Gly Ile Phe Gly Asn Gly Thr
    130                 135                 140
Glu Thr Cys His Thr Leu Pro His Ser Ala Thr Arg Ala Ala Met Leu
145                 150                 155                 160
Val Arg Ile Asn Thr Leu Leu Gln Gly Tyr Ser Gly Ile Arg Phe Glu
                165                 170                 175
Ile Leu Glu Ala Ile Thr Arg Leu Leu Asn Asn Asn Ile Thr Pro Cys
            180                 185                 190
Leu Pro Leu Arg Gly Thr Ile Thr Ala Ser Gly Asp Leu Val Pro Leu
        195                 200                 205
Ser Tyr Ile Ala Gly Leu Leu Thr Gly Arg Pro Asn Ser Lys Ala Thr
    210                 215                 220
Gly Pro Thr Gly Glu Val Leu Asp Ala Ala Glu Ala Phe Lys Ala Ala
225                 230                 235                 240
Gly Ile Glu Ser Gly Phe Phe Glu Leu Gln Pro Lys Glu Gly Leu Ala
                245                 250                 255
Leu Val Asn Gly Thr Ala Val Gly Ser Gly Leu Ala Ser Met Val Leu
            260                 265                 270
Phe Glu Thr Asn Val Leu Ala Val Leu Ser Glu Leu Leu Ser Ala Ile
        275                 280                 285
Phe Ala Glu Val Met Asn Gly Lys Pro Glu Phe Thr Asp His Leu Thr
    290                 295                 300
His Lys Leu Lys His His Pro Gly Gln Ile Glu Ala Ala Ala Ile Met
305                 310                 315                 320
Glu His Ile Leu Asp Gly Ser Ala Tyr Met Lys Ala Ala Lys Lys Leu
                325                 330                 335
His Glu Thr Asp Pro Leu Gln Lys Pro Lys Gln Asp Arg Tyr Ala Leu
            340                 345                 350

Arg Thr Ser Pro Gln Trp Leu Gly Pro Gln Ile Glu Val Ile Arg Phe
            355                 360                 365
Ser Thr Lys Ser Ile Glu Arg Glu Ile Asn Ser Val Asn Asp Asn Pro
    370                 375                 380
Leu Ile Asp Val Ser Arg Asn Lys Ala Ile His Gly Gly Asn Phe Gln
385                 390                 395                 400
Gly Thr Pro Ile Gly Val Ser Met Asp Asn Val Arg Leu Ala Ile Ala
                405                 410                 415
Ser Ile Gly Lys Leu Leu Phe Ala Gln Phe Ser Glu Leu Val Asn Asp
            420                 425                 430
Phe Tyr Asn Asn Gly Leu Pro Ser Asn Leu Thr Ala Ser Arg Asn Pro
        435                 440                 445
Ser Leu Asp Tyr Gly Phe Lys Gly Ala Glu Ile Ala Met Ala Ser Tyr
    450                 455                 460
Cys Ser Glu Leu Gln Tyr Leu Ala Asn Pro Val Thr Thr His Val Gln
465                 470                 475                 480
Ser Ala Glu Gln His Asn Gln Asp Val Asn Ser Leu Gly Leu Ile Ser
                485                 490                 495
Ser Arg Lys Thr Ala Glu Ala Val Asp Ile Leu Lys Leu Met Ser Thr
            500                 505                 510
Thr Phe Leu Val Ala Leu Cys Gln Ala Ile Asp Leu Arg His Leu Glu
        515                 520                 525
Glu Asn Leu Lys Ser Ala Val Lys Asn Thr Val Ser Gln Val Ser Lys
    530                 535                 540
Arg Val Leu Thr Thr Gly Ala Asn Gly Glu Leu His Pro Ser Arg Phe
545                 550                 555                 560
Cys Glu Lys Glu Leu Leu Lys Val Val Asp Arg Glu Tyr Val Phe Ala
                565                 570                 575
Tyr Val Asp Asp Pro Cys Ser Ala Thr Tyr Pro Leu Met Gln Lys Leu
            580                 585                 590
Arg Gln Val Phe Val Asp His Ala Leu Glu Asn Gly Glu Asn Glu Lys
        595                 600                 605
Asn Phe Ser Thr Ser Val Phe Gln Lys Ile Glu Ala Phe Glu Glu Glu
    610                 615                 620
Leu Lys Ala Leu Leu Pro Lys Glu Val Glu Ser Ala Arg Ala Ala Tyr
625                 630                 635                 640
Asp Ser Gly Asn Ser Ala Ile Asp Asn Lys Ile Lys Glu Cys Arg Ser
                645                 650                 655
Tyr Pro Leu Tyr Lys Phe Val Arg Glu Glu Leu Gly Thr Val Leu Leu
            660                 665                 670
Thr Gly Glu Lys Val Gln Ser Pro Gly Glu Glu Phe Asp Lys Val Phe
        675                 680                 685
Thr Ala Met Cys Gln Gly Lys Ile Ile Asp Pro Met Leu Glu Cys Leu
    690                 695                 700
Gly Glu Trp Asn Gly Ser Pro Leu Pro Ile Cys
705                 710                 715

<210> 2
<211> 718
<212> PRT
<213> Petroselinum crispum

<400> 2
Met Ala Tyr Val Asn Gly Thr Thr Asn Gly His Ala Asn Gly Asn Gly
1               5                   10                  15
Leu Asp Leu Cys Met Lys Lys Glu Asp Pro Leu Asn Trp Gly Val Ala
            20                  25                  30
Ala Glu Ala Leu Thr Gly Ser His Leu Asp Glu Val Lys Arg Met Val
        35                  40                  45
Ala Glu Tyr Arg Lys Pro Val Val Lys Leu Glu Gly Glu Thr Leu Thr
    50                  55                  60
Ile Ser Gln Val Ala Ala Ile Ser Ala Arg Asp Asp Ser Gly Val Lys

65                  70                  75                  80
Val Glu Leu Ser Glu Glu Ala Arg Ala Gly Val Lys Ala Ser Ser Asp
                85                  90                  95
Trp Val Met Asp Ser Met Asn Lys Gly Thr Asp Ser Tyr Gly Val Thr
            100                 105                 110
Thr Gly Phe Gly Ala Thr Ser His Arg Arg Thr Lys Gln Gly Gly Ala
        115                 120                 125
Leu Gln Lys Glu Leu Ile Arg Phe Leu Asn Ala Gly Ile Phe Gly Ser
    130                 135                 140
Gly Ala Glu Ala Gly Asn Asn Thr Leu Pro His Ser Ala Thr Arg Ala
145                 150                 155                 160
Ala Met Leu Val Arg Ile Asn Thr Leu Leu Gln Gly Tyr Ser Gly Ile
                165                 170                 175
Arg Phe Glu Ile Leu Glu Ala Ile Thr Lys Phe Leu Asn His Asn Ile
            180                 185                 190
Thr Pro Cys Leu Pro Leu Arg Gly Thr Ile Thr Ala Ser Gly Asp Leu
        195                 200                 205
Val Pro Leu Ser Tyr Ile Ala Gly Leu Leu Thr Gly Arg Pro Asn Ser
    210                 215                 220
Lys Ala Val Gly Pro Thr Gly Val Thr Leu Ser Pro Glu Glu Ala Phe
225                 230                 235                 240
Lys Leu Ala Gly Val Glu Gly Gly Phe Phe Glu Leu Gln Pro Lys Glu
                245                 250                 255
Gly Leu Ala Leu Val Asn Gly Thr Ala Val Gly Ser Gly Met Ala Ser
            260                 265                 270
Met Val Leu Phe Glu Ala Asn Ile Leu Ala Val Leu Ala Glu Val Met
        275                 280                 285
Ser Ala Ile Phe Ala Glu Val Met Gln Gly Lys Pro Glu Phe Thr Asp
    290                 295                 300
His Leu Thr His Lys Leu Lys His His Pro Gly Gln Ile Glu Ala Ala
305                 310                 315                 320
Ala Ile Met Glu His Ile Leu Asp Gly Ser Ala Tyr Val Lys Ala Ala
                325                 330                 335
Gln Lys Leu His Glu Met Asp Pro Leu Gln Lys Pro Lys Gln Asp Arg
            340                 345                 350
Tyr Ala Leu Arg Thr Ser Pro Gln Trp Leu Gly Pro Gln Ile Glu Val
        355                 360                 365
Ile Arg Ser Ser Thr Lys Met Ile Glu Arg Glu Ile Asn Ser Val Asn
    370                 375                 380
Asp Asn Pro Leu Ile Asp Val Ser Arg Asn Lys Ala Ile His Gly Gly
385                 390                 395                 400
Asn Phe Gln Gly Ser Pro Ile Gly Val Ser Met Asp Asn Thr Arg Leu
                405                 410                 415
Ala Ile Ala Ala Ile Gly Lys Leu Met Phe Ala Gln Phe Ser Glu Leu
            420                 425                 430
Val Asn Asp Phe Tyr Asn Asn Gly Leu Pro Ser Asn Leu Ser Gly Gly
        435                 440                 445
Arg Asn Pro Ser Leu Asp Tyr Gly Phe Lys Gly Ala Glu Ile Ala Met
    450                 455                 460
Ala Ser Tyr Cys Ser Glu Leu Gln Phe Leu Ala Asn Pro Val Thr Asn
465                 470                 475                 480
His Val Gln Ser Ala Glu Gln His Asn Gln Asp Val Asn Ser Leu Gly
                485                 490                 495
Leu Ile Ser Ser Arg Lys Thr Ser Glu Ala Val Glu Ile Leu Lys Leu
            500                 505                 510
Met Ser Thr Thr Phe Leu Val Gly Leu Cys Gln Ala Ile Asp Leu Arg
        515                 520                 525
His Leu Glu Glu Asn Leu Lys Ser Thr Val Lys Asn Thr Val Ser Gln
    530                 535                 540
Val Ala Lys Arg Val Leu Thr Met Gly Val Asn Gly Glu Leu His Pro
545                 550                 555                 560
Ser Arg Phe Cys Glu Lys Asp Leu Leu Arg Val Val Asp Arg Glu Tyr
                565                 570                 575

Ile Phe Ala Tyr Ile Asp Asp Pro Cys Ser Ala Thr Tyr Pro Leu Met
            580                 585                 590
Gln Lys Leu Arg Glu Thr Leu Val Glu His Ala Leu Asn Asn Gly Asp
        595                 600                 605
Lys Glu Arg Asn Leu Ser Thr Ser Ile Phe Gln Lys Ile Ala Ala Phe
    610                 615                 620
Glu Asp Glu Leu Lys Ala Leu Leu Pro Lys Glu Val Glu Thr Ala Arg
625                 630                 635                 640
Ala Ala Leu Glu Ser Gly Asn Pro Ala Ile Pro Asn Arg Ile Lys Glu
                645                 650                 655
Cys Arg Ser Tyr Pro Leu Tyr Lys Phe Val Arg Glu Glu Leu Gly Thr
            660                 665                 670
Glu Tyr Leu Thr Gly Glu Lys Val Arg Ser Pro Gly Glu Glu Phe Glu
        675                 680                 685
Lys Val Phe Thr Ala Met Ser Lys Gly Glu Ile Ile Asp Pro Leu Leu
    690                 695                 700
Glu Cys Leu Glu Ser Trp Asn Gly Ala Pro Leu Pro Ile Cys
705                 710                 715

<210> 3
<211> 506
<212> PRT
<213> Glycine max

<400> 3
Met Asp Leu Leu Leu Leu Glu Lys Thr Leu Ile Gly Leu Phe Leu Ala
1               5                   10                  15
Ala Val Val Ala Ile Ala Val Ser Thr Leu Arg Gly Arg Lys Phe Lys
            20                  25                  30
Leu Pro Pro Gly Pro Leu Pro Val Pro Ile Phe Gly Asn Trp Leu Gln
        35                  40                  45
Val Gly Asp Asp Leu Asn His Arg Asn Leu Thr Asp Leu Ala Lys Lys
    50                  55                  60
Phe Gly Asp Ile Phe Leu Leu Arg Met Gly Gln Arg Asn Leu Val Val
65                  70                  75                  80
Val Ser Ser Pro Glu Leu Ala Lys Glu Val Leu His Thr Gln Gly Val
                85                  90                  95
Glu Phe Gly Ser Arg Thr Arg Asn Val Val Phe Asp Ile Phe Thr Gly
            100                 105                 110
Lys Gly Gln Asp Met Val Phe Thr Val Tyr Gly Glu His Trp Arg Lys
        115                 120                 125
Met Arg Arg Ile Met Thr Val Pro Phe Phe Thr Asn Lys Val Val Gln
    130                 135                 140
Gln Tyr Arg His Gly Trp Glu Ser Glu Ala Ala Ala Val Val Glu Asp
145                 150                 155                 160
Val Lys Lys Asn Pro Asp Ala Ala Val Ser Gly Thr Val Ile Arg Arg
                165                 170                 175
Arg Leu Gln Leu Met Met Tyr Asn Asn Met Tyr Arg Ile Met Phe Asp
            180                 185                 190
Arg Arg Phe Glu Ser Glu Glu Asp Pro Ile Phe Gln Arg Leu Arg Ala
        195                 200                 205
Leu Asn Gly Glu Arg Ser Arg Leu Ala Gln Ser Phe Glu Tyr Asn Tyr
    210                 215                 220
Gly Asp Phe Ile Pro Ile Leu Arg Pro Phe Leu Lys Gly Tyr Leu Lys
225                 230                 235                 240
Ile Cys Lys Glu Val Lys Glu Thr Arg Leu Lys Leu Phe Lys Asp Tyr
                245                 250                 255
Phe Val Asp Glu Arg Lys Lys Leu Gly Ser Thr Lys Ser Thr Asn Asn
            260                 265                 270
Asn Asn Glu Leu Lys Cys Ala Ile Asp His Ile Leu Asp Ala Gln Arg
        275                 280                 285
Lys Gly Glu Ile Asn Glu Asp Asn Val Leu Tyr Ile Val Glu Asn Ile

290                 295                 300
Asn Val Ala Ala Ile Glu Thr Thr Leu Trp Ser Ile Glu Trp Gly Ile
305                 310                 315                 320
Ala Glu Leu Val Asn His Pro Glu Ile Gln Gln Lys Leu Arg Asp Glu
                325                 330                 335
Ile Asp Arg Val Leu Gly Ala Gly His Gln Val Thr Glu Pro Asp Ile
            340                 345                 350
Gln Lys Leu Pro Tyr Leu Gln Ala Val Val Lys Glu Thr Leu Arg Leu
        355                 360                 365
Arg Met Ala Ile Pro Leu Leu Val Pro His Met Asn Leu His Asp Ala
    370                 375                 380
Lys Leu Gly Gly Tyr Asp Ile Pro Ala Glu Ser Lys Ile Leu Val Asn
385                 390                 395                 400
Ala Trp Trp Leu Ala Asn Asn Pro Ala His Trp Lys Lys Pro Glu Glu
                405                 410                 415
Phe Arg Pro Glu Arg Phe Phe Glu Glu Glu Ser Leu Val Glu Ala Asn
            420                 425                 430
Gly Asn Asp Phe Arg Tyr Leu Pro Phe Gly Val Gly Arg Arg Ser Cys
        435                 440                 445
Pro Gly Ile Ile Leu Ala Leu Pro Ile Leu Gly Ile Thr Leu Gly Arg
    450                 455                 460
Leu Val Gln Asn Phe Glu Leu Leu Pro Pro Pro Gly Gln Ser Gln Ile
465                 470                 475                 480
Asp Thr Ser Glu Lys Gly Gly Gln Phe Ser Leu His Ile Leu Lys His
                485                 490                 495
Ser Thr Ile Val Ala Lys Pro Arg Ser Phe
            500                 505

<210> 4
<211> 506
<212> PRT
<213> Petroselinum crispum

<400> 4
Met Met Asp Phe Val Leu Leu Glu Lys Ala Leu Leu Gly Leu Phe Ile
1               5                   10                  15
Ala Thr Ile Val Ala Ile Thr Ile Ser Lys Leu Arg Gly Lys Lys Leu
            20                  25                  30
Lys Leu Pro Pro Gly Pro Ile Pro Val Pro Val Phe Gly Asn Trp Leu
        35                  40                  45
Gln Val Gly Asp Asp Leu Asn Gln Arg Asn Leu Val Asp Tyr Ala Lys
    50                  55                  60
Lys Phe Gly Asp Leu Phe Met Leu Arg Met Gly Gln Arg Asn Leu Val
65                  70                  75                  80
Val Val Ser Ser Pro Glu Leu Ala Lys Asp Val Leu His Thr Gln Gly
                85                  90                  95
Val Glu Phe Gly Ser Arg Thr Arg Asn Val Val Phe Asp Ile Phe Thr
            100                 105                 110
Gly Lys Gly Gln Asp Met Val Phe Thr Val Tyr Ser Glu His Trp Arg
        115                 120                 125
Lys Met Arg Arg Ile Met Thr Val Pro Phe Phe Thr Asn Lys Val Val
    130                 135                 140
Gln Gln Tyr Arg Phe Gly Trp Glu Asp Glu Ala Ala Arg Val Val Glu
145                 150                 155                 160
Asp Val Lys Ala Asn Pro Glu Ala Ala Thr Asn Gly Ile Val Leu Arg
                165                 170                 175
Asn Arg Leu Gln Leu Leu Met Tyr Asn Asn Met Tyr Arg Ile Met Phe
            180                 185                 190
Asp Arg Arg Phe Glu Ser Val Asp Asp Pro Leu Phe Leu Lys Leu Lys
        195                 200                 205
Ala Leu Asn Gly Glu Arg Ser Arg Leu Ala Gln Ser Phe Glu Tyr His
    210                 215                 220

Phe Gly Asp Phe Ile Pro Ile Leu Arg Pro Phe Leu Arg Gly Tyr Leu
225                 230                 235                 240
Lys Leu Cys Gln Glu Ile Lys Asp Lys Arg Leu Lys Leu Phe Lys Asp
                245                 250                 255
Tyr Phe Val Asp Glu Arg Lys Lys Leu Glu Ser Ile Lys Ser Val Asp
            260                 265                 270
Asn Asn Ser Leu Lys Cys Ala Ile Asp His Ile Ile Glu Ala Gln Gln
        275                 280                 285
Lys Gly Glu Ile Asn Glu Asp Asn Val Leu Tyr Ile Val Glu Asn Ile
    290                 295                 300
Asn Val Ala Ala Ile Glu Thr Thr Leu Trp Ser Ile Glu Trp Gly Ile
305                 310                 315                 320
Ala Glu Leu Val Asn Asn Pro Glu Ile Gln Lys Lys Leu Arg His Glu
                325                 330                 335
Leu Asp Thr Val Leu Gly Ala Gly Val Gln Ile Cys Glu Pro Asp Val
            340                 345                 350
Gln Lys Leu Pro Tyr Leu Gln Ala Val Ile Lys Glu Thr Leu Arg Tyr
        355                 360                 365
Arg Met Ala Ile Pro Leu Leu Val Pro His Met Asn Leu His Asp Ala
    370                 375                 380
Lys Leu Ala Gly Tyr Asp Ile Pro Ala Glu Ser Lys Ile Leu Val Asn
385                 390                 395                 400
Ala Trp Trp Leu Ala Asn Asn Pro Ala His Trp Asn Lys Pro Asp Glu
                405                 410                 415
Phe Arg Pro Glu Arg Phe Leu Glu Glu Glu Ser Lys Val Glu Ala Asn
            420                 425                 430
Gly Asn Asp Phe Lys Tyr Ile Pro Phe Gly Val Gly Arg Arg Ser Cys
        435                 440                 445
Pro Gly Ile Ile Leu Ala Leu Pro Ile Leu Gly Ile Val Ile Gly Arg
    450                 455                 460
Leu Val Gln Asn Phe Glu Leu Leu Pro Pro Pro Gly Gln Ser Lys Ile
465                 470                 475                 480
Asp Thr Ala Glu Lys Gly Gly Gln Phe Ser Leu Gln Ile Leu Lys His
                485                 490                 495
Ser Thr Ile Val Cys Lys Pro Arg Ser Leu
            500                 505

<210> 5
<211> 570
<212> PRT
<213> Populus tremuloides

<400> 5
Met Met Ser Val Ala Thr Val Glu Pro Pro Lys Pro Glu Leu Ser Pro
1               5                   10                  15
Pro Gln Asn Gln Asn Ala Pro Ser Ser His Glu Thr Asp His Ile Phe
            20                  25                  30
Arg Ser Lys Leu Pro Asp Ile Thr Ile Ser Asn Asp Leu Pro Leu His
        35                  40                  45
Ala Tyr Cys Phe Glu Asn Leu Ser Asp Phe Ser Asp Arg Pro Cys Leu
    50                  55                  60
Ile Ser Gly Ser Thr Gly Lys Thr Tyr Ser Phe Ala Glu Thr His Leu
65                  70                  75                  80
Ile Ser Arg Lys Val Ala Ala Gly Leu Ser Asn Leu Gly Ile Lys Lys
                85                  90                  95
Gly Asp Val Ile Met Thr Leu Leu Gln Asn Cys Pro Glu Phe Val Phe
            100                 105                 110
Ser Phe Ile Gly Ala Ser Met Ile Gly Ala Val Ile Thr Thr Ala Asn
        115                 120                 125
Pro Phe Tyr Thr Gln Ser Glu Ile Phe Lys Gln Phe Ser Ala Ser Arg
    130                 135                 140
Ala Lys Leu Ile Ile Thr Gln Ser Gln Tyr Val Asn Lys Leu Gly Asp

145                 150                 155                 160
Ser Asp Cys His Glu Asn Asn Gln Lys Pro Gly Glu Asp Phe Ile Val
                165                 170                 175
Ile Thr Ile Asp Asp Pro Pro Glu Asn Cys Leu His Phe Asn Val Leu
            180                 185                 190
Val Glu Ala Ser Glu Ser Glu Met Pro Thr Val Ser Ile Leu Pro Asp
        195                 200                 205
Asp Pro Val Ala Leu Pro Phe Ser Ser Gly Thr Thr Gly Leu Pro Lys
    210                 215                 220
Gly Val Ile Leu Thr His Lys Ser Leu Ile Thr Ser Val Ala Gln Gln
225                 230                 235                 240
Val Asp Gly Glu Ile Pro Asn Leu Tyr Leu Lys Gln Asp Asp Val Val
                245                 250                 255
Leu Cys Val Leu Pro Leu Phe His Ile Phe Ser Leu Asn Ser Val Leu
            260                 265                 270
Leu Cys Ser Leu Arg Ala Gly Ser Ala Val Leu Leu Met Gln Lys Phe
        275                 280                 285
Glu Ile Gly Ser Leu Leu Glu Leu Ile Gln Lys His Asn Val Ser Val
    290                 295                 300
Ala Ala Val Val Pro Pro Leu Val Leu Ala Leu Ala Lys Asn Pro Leu
305                 310                 315                 320
Glu Ala Asn Phe Asp Leu Ser Ser Ile Arg Val Val Leu Ser Gly Ala
                325                 330                 335
Ala Pro Leu Gly Lys Glu Leu Glu Asp Ala Leu Arg Ser Arg Val Pro
            340                 345                 350
Gln Ala Ile Leu Gly Gln Gly Tyr Gly Met Thr Glu Ala Gly Pro Val
        355                 360                 365
Leu Ser Met Cys Leu Ala Phe Ser Lys Gln Pro Phe Pro Thr Lys Ser
    370                 375                 380
Gly Ser Cys Gly Thr Val Val Arg Asn Ala Glu Leu Lys Val Ile Asp
385                 390                 395                 400
Pro Glu Thr Gly Arg Ser Leu Gly Tyr Asn Gln Pro Gly Glu Ile Cys
                405                 410                 415
Ile Arg Gly Ser Gln Ile Met Lys Gly Tyr Leu Asn Asp Ala Glu Ala
            420                 425                 430
Thr Ala Asn Thr Ile Asp Val Glu Gly Trp Leu His Thr Gly Asp Ile
        435                 440                 445
Gly Tyr Val Asp Asp Asp Asp Glu Ile Phe Ile Val Asp Arg Val Lys
    450                 455                 460
Glu Ile Ile Lys Phe Lys Gly Phe Gln Val Pro Pro Ala Glu Leu Glu
465                 470                 475                 480
Ala Leu Leu Val Asn His Pro Ser Ile Ala Asp Ala Ala Val Val Pro
                485                 490                 495
Gln Lys Asp Glu Val Ala Gly Glu Val Pro Val Ala Phe Val Val Arg
            500                 505                 510
Ser Asp Asp Leu Asp Leu Ser Glu Glu Ala Val Lys Glu Tyr Ile Ala
        515                 520                 525
Lys Gln Val Val Phe Tyr Lys Lys Leu His Lys Val Phe Phe Val His
    530                 535                 540
Ser Ile Pro Lys Ser Ala Ser Gly Lys Ile Leu Arg Lys Asp Leu Arg
545                 550                 555                 560
Ala Lys Leu Ala Thr Ala Thr Thr Met Ser
                565                 570

<210> 6
<211> 276
<212> PRT
<213> Pseudomonas fluorescens

<400> 6
Met Ser Asn Tyr Glu Gly Arg Trp Thr Thr Val Lys Val Glu Ile Glu
1               5                   10                  15

Asp Gly Ile Ala Trp Val Ile Leu Asn Arg Pro Glu Lys Arg Asn Ala
            20                  25                  30
Met Ser Pro Thr Leu Asn Arg Glu Met Ile Asp Val Leu Glu Thr Leu
        35                  40                  45
Glu Gln Asp Pro Ala Ala Gly Val Leu Val Leu Thr Gly Ala Gly Glu
    50                  55                  60
Ala Trp Thr Ala Gly Met Asp Leu Lys Glu Tyr Phe Arg Glu Val Asp
65                  70                  75                  80
Ala Gly Pro Glu Ile Leu Gln Glu Lys Ile Arg Arg Glu Ala Ser Gln
                85                  90                  95
Trp Gln Trp Lys Leu Leu Arg Met Tyr Ala Lys Pro Thr Ile Ala Met
            100                 105                 110
Val Asn Gly Trp Cys Phe Gly Gly Gly Phe Ser Pro Leu Val Ala Cys
        115                 120                 125
Asp Leu Ala Ile Cys Ala Asp Glu Ala Thr Phe Gly Leu Ser Glu Ile
    130                 135                 140
Asn Trp Gly Ile Pro Pro Gly Asn Leu Val Ser Lys Ala Met Ala Asp
145                 150                 155                 160
Thr Val Gly His Arg Gln Ser Leu Tyr Tyr Ile Met Thr Gly Lys Thr
                165                 170                 175
Phe Gly Gly Gln Lys Ala Ala Glu Met Gly Leu Val Asn Asp Ser Val
            180                 185                 190
Pro Leu Ala Arg Leu Arg Glu Val Thr Ile Glu Leu Ala Arg Asn Leu
        195                 200                 205
Leu Glu Lys Asn Pro Val Val Leu Arg Ala Ala Lys His Gly Phe Lys
    210                 215                 220
Arg Cys Arg Glu Leu Thr Trp Glu Gln Asn Glu Asp Tyr Leu Tyr Ala
225                 230                 235                 240
Lys Leu Asp Gln Ser Arg Leu Leu Asp Thr Glu Gly Gly Arg Glu Gln
                245                 250                 255
Gly Met Lys Gln Phe Leu Asp Asp Lys Ser Ile Lys Pro Gly Leu Gln
            260                 265                 270
Ala Tyr Lys Arg
        275

<210> 7
<211> 276
<212> PRT
<213> Azotobacter vinelandii

<400> 7
Met Asn Lys Tyr Glu Gly Arg Trp Lys Thr Val Ile Val Glu Ile Glu
1               5                   10                  15
Gly Gly Ile Ala Trp Val Thr Leu Asn Arg Pro Asp Lys Arg Asn Ala
            20                  25                  30
Met Ser Pro Thr Leu Asn Arg Glu Met Arg Asp Val Leu Glu Thr Leu
        35                  40                  45
Glu Gln Asp Pro Ala Ala Arg Val Leu Val Leu Thr Gly Ala Gly Ser
    50                  55                  60
Ala Trp Thr Ala Gly Met Asp Leu Lys Glu Tyr Phe Arg Glu Val Asp
65                  70                  75                  80
Ala Gly Pro Glu Ile Leu Gln Glu Lys Ile Arg Arg Glu Ala Cys Glu
                85                  90                  95
Trp Gln Trp Lys Leu Leu Arg Met Tyr Ala Lys Pro Thr Val Ala Met
            100                 105                 110
Val Asn Gly Trp Cys Phe Gly Gly Gly Phe Ser Pro Leu Val Ala Cys
        115                 120                 125
Asp Leu Ala Ile Cys Ala Asp Glu Ala Thr Phe Gly Leu Ser Glu Ile
    130                 135                 140
Asn Trp Gly Ile Pro Pro Gly Asn Leu Val Ser Lys Ala Met Ala Asp
145                 150                 155                 160
Thr Val Gly His Arg Gln Ala Leu Tyr Tyr Ile Met Thr Gly Lys Thr

165                 170                 175
Phe Asp Gly Arg Gln Ala Ala Glu Met Gly Leu Val Asn Gln Ser Val
            180                 185                 190
Pro Leu Ala Gln Leu Arg Glu Thr Val Ala Thr Leu Cys Gln Asp Leu
        195                 200                 205
Leu Asp Lys Asn Pro Val Val Leu Arg Ala Ala Lys Asn Gly Phe Lys
    210                 215                 220
Arg Cys Arg Glu Leu Thr Trp Glu Gln Asn Glu Asp Tyr Leu Tyr Ala
225                 230                 235                 240
Lys Leu Asp Gln Ser Arg Leu Leu Asp Glu Glu Gly Gly Arg Glu Glu
                245                 250                 255
Gly Met Arg Gln Phe Leu Asp Glu Lys Ser Ile Lys Pro Gly Leu Gln
            260                 265                 270
Ala Tyr Lys Arg
        275

<210> 8
<211> 394
<212> PRT
<213> Pseudomonas aeruginosa

<400> 8
Met Lys Thr Gln Val Ala Ile Ile Gly Ala Gly Pro Ser Gly Leu Leu
1               5                   10                  15
Leu Gly Gln Leu Leu His Lys Ala Gly Ile Asp Asn Val Ile Leu Glu
            20                  25                  30
Arg Gln Thr Pro Asp Tyr Val Leu Gly Arg Ile Arg Ala Gly Val Leu
        35                  40                  45
Glu Gln Gly Met Val Asp Leu Leu Arg Glu Ala Gly Val Asp Arg Arg
    50                  55                  60
Met Ala Arg Asp Gly Leu Val His Glu Gly Val Glu Ile Ala Phe Ala
65                  70                  75                  80
Gly Gln Arg Arg Arg Ile Asp Leu Lys Arg Leu Ser Gly Gly Lys Thr
                85                  90                  95
Val Thr Val Tyr Gly Gln Thr Glu Val Thr Arg Asp Leu Met Glu Ala
            100                 105                 110
Arg Glu Ala Cys Gly Ala Thr Thr Val Tyr Gln Ala Ala Glu Val Arg
        115                 120                 125
Leu His Asp Leu Gln Gly Glu Arg Pro Tyr Val Thr Phe Glu Arg Asp
    130                 135                 140
Gly Glu Arg Leu Arg Leu Asp Cys Asp Tyr Ile Ala Gly Cys Asp Gly
145                 150                 155                 160
Phe His Gly Ile Ser Arg Gln Ser Ile Pro Ala Glu Arg Leu Lys Val
                165                 170                 175
Phe Glu Arg Val Tyr Pro Phe Gly Trp Leu Gly Leu Leu Ala Asp Thr
            180                 185                 190
Pro Pro Val Ser His Glu Leu Ile Tyr Ala Asn His Pro Arg Gly Phe
        195                 200                 205
Ala Leu Cys Ser Gln Arg Ser Ala Thr Arg Ser Arg Tyr Tyr Val Gln
    210                 215                 220
Val Pro Leu Thr Glu Lys Val Glu Asp Trp Ser Asp Glu Arg Phe Trp
225                 230                 235                 240
Thr Glu Leu Lys Ala Arg Leu Pro Ala Glu Val Ala Glu Lys Leu Val
                245                 250                 255
Thr Gly Pro Ser Leu Glu Lys Ser Ile Ala Pro Leu Arg Ser Phe Val
            260                 265                 270
Val Glu Pro Met Gln His Gly Arg Leu Phe Leu Ala Gly Asp Ala Ala
        275                 280                 285
His Ile Val Pro Pro Thr Gly Ala Lys Gly Leu Asn Leu Ala Ala Ser
    290                 295                 300
Asp Val Ser Thr Leu Tyr Arg Leu Leu Leu Lys Ala Tyr Arg Glu Gly
305                 310                 315                 320

Arg Gly Glu Leu Leu Glu Arg Tyr Ser Ala Ile Cys Leu Arg Arg Ile
                325                 330                 335
Trp Lys Ala Glu Arg Phe Ser Trp Trp Met Thr Ser Val Leu His Arg
            340                 345                 350
Phe Pro Asp Thr Asp Ala Phe Ser Gln Arg Ile Gln Gln Thr Glu Leu
        355                 360                 365
Glu Tyr Tyr Leu Gly Ser Glu Ala Gly Leu Ala Thr Ile Ala Glu Asn
    370                 375                 380
Tyr Val Gly Leu Pro Tyr Glu Glu Ile Glu
385                 390

<210> 9
<211> 394
<212> PRT
<213> Azotobacter vinelandii

<400> 9
Met Lys Thr Gln Val Ala Ile Ile Gly Ala Gly Pro Ser Gly Leu Leu
1               5                   10                  15
Leu Gly Gln Leu Leu His Lys Ala Gly Ile Asp Asn Val Ile Leu Glu
            20                  25                  30
Arg His Ser Pro Asp Tyr Val Leu Gly Arg Ile Arg Ala Gly Val Leu
        35                  40                  45
Glu Gln Gly Val Val Asp Leu Leu Arg Glu Ala Gly Val Ala Glu Arg
    50                  55                  60
Met Asp Arg Glu Gly Leu Val His Glu Gly Ile Glu Leu Ala Cys Ser
65                  70                  75                  80
Gly Arg Arg Ile Arg Leu Asp Leu Lys Ala Leu Ser Gly Gly Lys Thr
                85                  90                  95
Val Met Val Tyr Gly Gln Thr Glu Val Thr Arg Asp Leu Met Asp Ala
            100                 105                 110
Arg Arg Ala Ser Gly Ala Pro Ile Val Tyr Glu Ala Gln Asn Val Arg
        115                 120                 125
Leu Ser Gly Leu Lys Asp Gly Met Pro His Val Thr Tyr Glu Lys Asp
    130                 135                 140
Gly Gln Thr His Arg Leu Asp Cys Asp Tyr Ile Ala Gly Cys Asp Gly
145                 150                 155                 160
Phe His Gly Val Ser Arg Gln Ser Ile Pro Ala Glu Ala Leu Ser His
                165                 170                 175
Tyr Glu Arg Val Tyr Pro Phe Gly Trp Leu Gly Leu Leu Ser Asp Thr
            180                 185                 190
Pro Pro Val His Glu Glu Leu Ile Tyr Ala His Thr Asp Leu Gly Phe
        195                 200                 205
Val Leu Cys Ser Gln Arg Ser Pro Thr Arg Ser Arg Tyr Tyr Leu Gln
    210                 215                 220
Val Pro Leu Ser Asp Arg Val Glu Asp Trp Ser Asp Glu Arg Phe Trp
225                 230                 235                 240
Asn Glu Leu Lys Arg Arg Leu Pro Gly Asp Val Ala Asn Arg Leu Val
                245                 250                 255
Thr Gly Pro Ser Leu Glu Lys Ser Ile Ala Pro Leu Arg Ser Tyr Val
            260                 265                 270
Val Glu Pro Met Gln Tyr Gly Arg Leu Phe Leu Val Gly Asp Ala Ala
        275                 280                 285
His Ile Val Pro Pro Thr Gly Ala Lys Gly Leu Asn Leu Ala Gly Ser
    290                 295                 300
Asp Val Cys Tyr Leu Tyr Arg Ile Leu Leu Lys Val Tyr Arg Glu Gly
305                 310                 315                 320
Arg Thr Glu Leu Leu Glu Lys Tyr Ser Glu Leu Ala Leu Arg Arg Val
                325                 330                 335
Trp Lys Gly Glu Arg Phe Ser Trp Phe Met Thr Asn Leu Leu His Asp
            340                 345                 350
Phe Glu Gly Ser Asp Ala Phe Asp Arg Arg Met Gln Leu Ala Asp Arg

355                 360                 365
Asp Tyr Tyr Leu Asp Ser Glu Ala Gly Arg Val Thr Ile Ala Glu Asn
    370                 375                 380
Tyr Val Gly Leu Pro Tyr Glu Glu Ile Ala
385                 390

<210> 10
<211> 365
<212> PRT
<213> Medicago sativa

<400> 10
Met Gly Ser Thr Gly Glu Thr Gln Ile Thr Pro Thr His Ile Ser Asp
1               5                   10                  15
Glu Glu Ala Asn Leu Phe Ala Met Gln Leu Ala Ser Ala Ser Val Leu
            20                  25                  30
Pro Met Ile Leu Lys Ser Ala Leu Glu Leu Asp Leu Leu Glu Ile Ile
        35                  40                  45
Ala Lys Ala Gly Pro Gly Ala Gln Ile Ser Pro Ile Glu Ile Ala Ser
    50                  55                  60
Gln Leu Pro Thr Thr Asn Pro Asp Ala Pro Val Met Leu Asp Arg Met
65                  70                  75                  80
Leu Arg Leu Leu Ala Cys Tyr Asn Ile Leu Thr Cys Ser Val Arg Thr
                85                  90                  95
Gln Gln Asp Gly Lys Val Gln Arg Leu Tyr Gly Leu Ala Thr Val Ala
            100                 105                 110
Lys Tyr Leu Val Lys Asn Glu Asp Gly Val Ser Ile Ser Ala Leu Asn
        115                 120                 125
Leu Met Asn Gln Asp Lys Val Leu Met Glu Ser Trp Tyr His Leu Lys
    130                 135                 140
Asp Ala Val Leu Asp Gly Gly Ile Pro Phe Asn Lys Ala Tyr Gly Met
145                 150                 155                 160
Thr Ala Phe Glu Tyr His Gly Thr Asp Pro Arg Phe Asn Lys Val Phe
                165                 170                 175
Asn Lys Gly Met Ser Asp His Ser Thr Ile Thr Met Lys Lys Ile Leu
            180                 185                 190
Glu Thr Tyr Thr Gly Phe Glu Gly Leu Lys Ser Leu Val Asp Val Gly
        195                 200                 205
Gly Gly Thr Gly Ala Val Ile Asn Thr Ile Val Ser Lys Tyr Pro Thr
    210                 215                 220
Ile Lys Gly Ile Asn Phe Asp Leu Pro His Val Ile Glu Asp Ala Pro
225                 230                 235                 240
Ser Tyr Pro Gly Val Glu His Val Gly Gly Asp Met Phe Val Ser Ile
                245                 250                 255
Pro Lys Ala Asp Ala Val Phe Met Lys Trp Ile Cys His Asp Trp Ser
            260                 265                 270
Asp Glu His Cys Leu Lys Phe Leu Lys Asn Cys Tyr Glu Ala Leu Pro
        275                 280                 285
Asp Asn Gly Lys Val Ile Val Ala Glu Cys Ile Leu Pro Val Ala Pro
    290                 295                 300
Asp Ser Ser Leu Ala Thr Lys Gly Val Val His Ile Asp Val Ile Met
305                 310                 315                 320
Leu Ala His Asn Pro Gly Gly Lys Glu Arg Thr Gln Lys Glu Phe Glu
                325                 330                 335
Asp Leu Ala Lys Gly Ala Gly Phe Gln Gly Phe Lys Val His Cys Asn
            340                 345                 350
Ala Phe Asn Thr Tyr Ile Met Glu Phe Leu Lys Lys Val
        355                 360                 365

<210> 11
<211> 365
<212> PRT

<213> Vanilla planifolia

<400> 11
Met Ala Thr Trp Val Glu His Gln Gln Gln Gln Asn Gly Ser Lys Asp
1               5                   10                  15
Val Asp Glu Glu Ala Cys Met Tyr Ala Met Gln Leu Ser Ser Met Val
            20                  25                  30
Val Leu Pro Met Thr Leu Arg Val Ala Val Glu Leu Gly Ile Leu Glu
        35                  40                  45
Gln Ile Gln Ala Gly Gly Pro Asp Ser Tyr Leu Thr Ala Glu Asp Leu
    50                  55                  60
Ala Ala Arg Leu Gly Asn Ser Asn Pro Leu Ala Pro Val Met Ile Glu
65                  70                  75                  80
Arg Ile Leu Arg Leu Leu Thr Ser Tyr Ser Ile Leu Asn Phe Thr Asp
                85                  90                  95
Thr Val Asp Gly Glu Gly Arg Thr Val Arg Ser Tyr Gly Ala Ala His
            100                 105                 110
Val Cys Lys Tyr Leu Thr Pro Asn Gln Asp Gly Val Ser Met Ala Pro
        115                 120                 125
Leu Val Leu Met Asn Thr Asp Lys Val Leu Met Glu Ser Trp Tyr His
    130                 135                 140
Met Lys Asp Ala Val Thr Asn Gly Gly Ile Pro Phe Asn Leu Ala Tyr
145                 150                 155                 160
Gly Met Thr Ala Phe Glu Tyr His Gly Lys Asp Leu Arg Phe Asn Lys
                165                 170                 175
Val Phe Asn Glu Gly Met Lys Asn Asn Ser Ile Ile Ile Thr Lys Lys
            180                 185                 190
Ile Leu Glu Arg Tyr Lys Arg Phe Glu Asp Val Asn Val Leu Ile Asp
        195                 200                 205
Val Gly Gly Gly Ile Gly Gly Thr Ile Ser Met Ile Thr Ala Lys Tyr
    210                 215                 220
Pro His Ile His Gly Ile Asn Phe Asp Leu Pro His Val Val Ser Glu
225                 230                 235                 240
Ala Pro Pro Phe Gln Gly Val Glu His Val Gly Gly Asn Met Phe Glu
                245                 250                 255
Ser Val Pro Ile Gly Asp Ala Ile Phe Ile Lys Trp Ile Leu His Asp
            260                 265                 270
Trp Ser Asp Glu His Cys Leu Lys Leu Leu Arg Asn Cys Ala Lys Ser
        275                 280                 285
Leu Pro Asp Lys Gly Lys Val Ile Val Val Glu Cys Ile Leu Pro Asp
    290                 295                 300
Ala Pro Leu Val Thr Pro Glu Ala Glu Gly Val Phe His Leu Asp Met
305                 310                 315                 320
Ile Met Leu Ala His Asn Pro Gly Gly Lys Glu Arg Thr Lys Lys Glu
                325                 330                 335
Phe Lys Glu Leu Ala Met Leu Ser Gly Phe Ser Asn Phe Lys Ala Leu
            340                 345                 350
Phe Ser Tyr Ala Asn Val Trp Val Met Glu Phe Asn Lys
        355                 360                 365

<210> 12
<211> 1174
<212> PRT
<213> Nocardia iowensis

<400> 12
Met Ala Val Asp Ser Pro Asp Glu Arg Leu Gln Arg Arg Ile Ala Gln
1               5                   10                  15
Leu Phe Ala Glu Asp Glu Gln Val Lys Ala Ala Arg Pro Leu Glu Ala
            20                  25                  30
Val Ser Ala Ala Val Ser Ala Pro Gly Met Arg Leu Ala Gln Ile Ala

35                  40                  45
Ala Thr Val Met Ala Gly Tyr Ala Asp Arg Pro Ala Ala Gly Gln Arg
    50                  55                  60
Ala Phe Glu Leu Asn Thr Asp Asp Ala Thr Gly Arg Thr Ser Leu Arg
65                  70                  75                  80
Leu Leu Pro Arg Phe Glu Thr Ile Thr Tyr Arg Glu Leu Trp Gln Arg
                85                  90                  95
Val Gly Glu Val Ala Ala Ala Trp His His Asp Pro Glu Asn Pro Leu
            100                 105                 110
Arg Ala Gly Asp Phe Val Ala Leu Leu Gly Phe Thr Ser Ile Asp Tyr
        115                 120                 125
Ala Thr Leu Asp Leu Ala Asp Ile His Leu Gly Ala Val Thr Val Pro
    130                 135                 140
Leu Gln Ala Ser Ala Ala Val Ser Gln Leu Ile Ala Ile Leu Thr Glu
145                 150                 155                 160
Thr Ser Pro Arg Leu Leu Ala Ser Thr Pro Glu His Leu Asp Ala Ala
                165                 170                 175
Val Glu Cys Leu Leu Ala Gly Thr Thr Pro Glu Arg Leu Val Val Phe
            180                 185                 190
Asp Tyr His Pro Glu Asp Asp Asp Gln Arg Ala Ala Phe Glu Ser Ala
        195                 200                 205
Arg Arg Arg Leu Ala Asp Ala Gly Ser Leu Val Ile Val Glu Thr Leu
    210                 215                 220
Asp Ala Val Arg Ala Arg Gly Arg Asp Leu Pro Ala Ala Pro Leu Phe
225                 230                 235                 240
Val Pro Asp Thr Asp Asp Asp Pro Leu Ala Leu Leu Ile Tyr Thr Ser
                245                 250                 255
Gly Ser Thr Gly Thr Pro Lys Gly Ala Met Tyr Thr Asn Arg Leu Ala
            260                 265                 270
Ala Thr Met Trp Gln Gly Asn Ser Met Leu Gln Gly Asn Ser Gln Arg
        275                 280                 285
Val Gly Ile Asn Leu Asn Tyr Met Pro Met Ser His Ile Ala Gly Arg
    290                 295                 300
Ile Ser Leu Phe Gly Val Leu Ala Arg Gly Gly Thr Ala Tyr Phe Ala
305                 310                 315                 320
Ala Lys Ser Asp Met Ser Thr Leu Phe Glu Asp Ile Gly Leu Val Arg
                325                 330                 335
Pro Thr Glu Ile Phe Phe Val Pro Arg Val Cys Asp Met Val Phe Gln
            340                 345                 350
Arg Tyr Gln Ser Glu Leu Asp Arg Arg Ser Val Ala Gly Ala Asp Leu
        355                 360                 365
Asp Thr Leu Asp Arg Glu Val Lys Ala Asp Leu Arg Gln Asn Tyr Leu
    370                 375                 380
Gly Gly Arg Phe Leu Val Ala Val Val Gly Ser Ala Pro Leu Ala Ala
385                 390                 395                 400
Glu Met Lys Thr Phe Met Glu Ser Val Leu Asp Leu Pro Leu His Asp
                405                 410                 415
Gly Tyr Gly Ser Thr Glu Ala Gly Ala Ser Val Leu Leu Asp Asn Gln
            420                 425                 430
Ile Gln Arg Pro Pro Val Leu Asp Tyr Lys Leu Val Asp Val Pro Glu
        435                 440                 445
Leu Gly Tyr Phe Arg Thr Asp Arg Pro His Pro Arg Gly Glu Leu Leu
    450                 455                 460
Leu Lys Ala Glu Thr Thr Ile Pro Gly Tyr Tyr Lys Arg Pro Glu Val
465                 470                 475                 480
Thr Ala Glu Ile Phe Asp Glu Asp Gly Phe Tyr Lys Thr Gly Asp Ile
                485                 490                 495
Val Ala Glu Leu Glu His Asp Arg Leu Val Tyr Val Asp Arg Arg Asn
            500                 505                 510
Asn Val Leu Lys Leu Ser Gln Gly Glu Phe Val Thr Val Ala His Leu
        515                 520                 525
Glu Ala Val Phe Ala Ser Ser Pro Leu Ile Arg Gln Ile Phe Ile Tyr
    530                 535                 540

Gly Ser Ser Glu Arg Ser Tyr Leu Leu Ala Val Ile Val Pro Thr Asp
545                 550                 555                 560
Asp Ala Leu Arg Gly Arg Asp Thr Ala Thr Leu Lys Ser Ala Leu Ala
                565                 570                 575
Glu Ser Ile Gln Arg Ile Ala Lys Asp Ala Asn Leu Gln Pro Tyr Glu
            580                 585                 590
Ile Pro Arg Asp Phe Leu Ile Glu Thr Glu Pro Phe Thr Ile Ala Asn
        595                 600                 605
Gly Leu Leu Ser Gly Ile Ala Lys Leu Leu Arg Pro Asn Leu Lys Glu
    610                 615                 620
Arg Tyr Gly Ala Gln Leu Glu Gln Met Tyr Thr Asp Leu Ala Thr Gly
625                 630                 635                 640
Gln Ala Asp Glu Leu Leu Ala Leu Arg Arg Glu Ala Ala Asp Leu Pro
                645                 650                 655
Val Leu Glu Thr Val Ser Arg Ala Ala Lys Ala Met Leu Gly Val Ala
            660                 665                 670
Ser Ala Asp Met Arg Pro Asp Ala His Phe Thr Asp Leu Gly Gly Asp
        675                 680                 685
Ser Leu Ser Ala Leu Ser Phe Ser Asn Leu Leu His Glu Ile Phe Gly
    690                 695                 700
Val Glu Val Pro Val Gly Val Val Val Ser Pro Ala Asn Glu Leu Arg
705                 710                 715                 720
Asp Leu Ala Asn Tyr Ile Glu Ala Glu Arg Asn Ser Gly Ala Lys Arg
                725                 730                 735
Pro Thr Phe Thr Ser Val His Gly Gly Gly Ser Glu Ile Arg Ala Ala
            740                 745                 750
Asp Leu Thr Leu Asp Lys Phe Ile Asp Ala Arg Thr Leu Ala Ala Ala
        755                 760                 765
Asp Ser Ile Pro His Ala Pro Val Pro Ala Gln Thr Val Leu Leu Thr
    770                 775                 780
Gly Ala Asn Gly Tyr Leu Gly Arg Phe Leu Cys Leu Glu Trp Leu Glu
785                 790                 795                 800
Arg Leu Asp Lys Thr Gly Gly Thr Leu Ile Cys Val Val Arg Gly Ser
                805                 810                 815
Asp Ala Ala Ala Ala Arg Lys Arg Leu Asp Ser Ala Phe Asp Ser Gly
            820                 825                 830
Asp Pro Gly Leu Leu Glu His Tyr Gln Gln Leu Ala Ala Arg Thr Leu
        835                 840                 845
Glu Val Leu Ala Gly Asp Ile Gly Asp Pro Asn Leu Gly Leu Asp Asp
    850                 855                 860
Ala Thr Trp Gln Arg Leu Ala Glu Thr Val Asp Leu Ile Val His Pro
865                 870                 875                 880
Ala Ala Leu Val Asn His Val Leu Pro Tyr Thr Gln Leu Phe Gly Pro
                885                 890                 895
Asn Val Val Gly Thr Ala Glu Ile Val Arg Leu Ala Ile Thr Ala Arg
            900                 905                 910
Arg Lys Pro Val Thr Tyr Leu Ser Thr Val Gly Val Ala Asp Gln Val
        915                 920                 925
Asp Pro Ala Glu Tyr Gln Glu Asp Ser Asp Val Arg Glu Met Ser Ala
    930                 935                 940
Val Arg Val Val Arg Glu Ser Tyr Ala Asn Gly Tyr Gly Asn Ser Lys
945                 950                 955                 960
Trp Ala Gly Glu Val Leu Leu Arg Glu Ala His Asp Leu Cys Gly Leu
                965                 970                 975
Pro Val Ala Val Phe Arg Ser Asp Met Ile Leu Ala His Ser Arg Tyr
            980                 985                 990
Ala Gly Gln Leu Asn Val Gln Asp Val Phe Thr Arg Leu Ile Leu Ser
        995                 1000                1005
Leu Val Ala Thr Gly Ile Ala Pro Tyr Ser Phe Tyr Arg Thr Asp Ala
    1010                1015                1020
Asp Gly Asn Arg Gln Arg Ala His Tyr Asp Gly Leu Pro Ala Asp Phe
1025                1030                1035                1040
Thr Ala Ala Ala Ile Thr Ala Leu Gly Ile Gln Ala Thr Glu Gly Phe

1045                1050                1055
Arg Thr Tyr Asp Val Leu Asn Pro Tyr Asp Asp Gly Ile Ser Leu Asp
        1060                1065                1070
Glu Phe Val Asp Trp Leu Val Glu Ser Gly His Pro Ile Gln Arg Ile
    1075                1080                1085
Thr Asp Tyr Ser Asp Trp Phe His Arg Phe Glu Thr Ala Ile Arg Ala
    1090                1095                1100
Leu Pro Glu Lys Gln Arg Gln Ala Ser Val Leu Pro Leu Leu Asp Ala
1105                1110                1115                1120
Tyr Arg Asn Pro Cys Pro Ala Val Arg Gly Ala Ile Leu Pro Ala Lys
                1125                1130                1135
Glu Phe Gln Ala Ala Val Gln Thr Ala Lys Ile Gly Pro Glu Gln Asp
        1140                1145                1150
Ile Pro His Leu Ser Ala Pro Leu Ile Asp Lys Tyr Val Ser Asp Leu
    1155                1160                1165
Glu Leu Leu Gln Leu Leu
    1170

<210> 13
<211> 222
<212> PRT
<213> Nocardia iowensis


<400> 13
Met Ile Glu Thr Ile Leu Pro Ala Gly Val Glu Ser Ala Glu Leu Leu
1               5                   10                  15
Glu Tyr Pro Glu Asp Leu Lys Ala His Pro Ala Glu Glu His Leu Ile
            20                  25                  30
Ala Lys Ser Val Glu Lys Arg Arg Arg Asp Phe Ile Gly Ala Arg His
        35                  40                  45
Cys Ala Arg Leu Ala Leu Ala Glu Leu Gly Glu Pro Pro Val Ala Ile
    50                  55                  60
Gly Lys Gly Glu Arg Gly Ala Pro Ile Trp Pro Arg Gly Val Val Gly
65                  70                  75                  80
Ser Leu Thr His Cys Asp Gly Tyr Arg Ala Ala Ala Val Ala His Lys
                85                  90                  95
Met Arg Phe Arg Ser Ile Gly Ile Asp Ala Glu Pro His Ala Thr Leu
            100                 105                 110
Pro Glu Gly Val Leu Asp Ser Val Ser Leu Pro Pro Glu Arg Glu Trp
        115                 120                 125
Leu Lys Thr Thr Asp Ser Ala Leu His Leu Asp Arg Leu Leu Phe Cys
    130                 135                 140
Ala Lys Glu Ala Thr Tyr Lys Ala Trp Trp Pro Leu Thr Ala Arg Trp
145                 150                 155                 160
Leu Gly Phe Glu Glu Ala His Ile Thr Phe Glu Ile Glu Asp Gly Ser
                165                 170                 175
Ala Asp Ser Gly Asn Gly Thr Phe His Ser Glu Leu Leu Val Pro Gly
            180                 185                 190
Gln Thr Asn Asp Gly Gly Thr Pro Leu Leu Ser Phe Asp Gly Arg Trp
        195                 200                 205
Leu Ile Ala Asp Gly Phe Ile Leu Thr Ala Ile Ala Tyr Ala
    210                 215                 220